# EUROPEAN PATENT SPECIFICATION

(11) **EP 4 015 739 B1**
(45) Date of publication and mention of the grant of the patent: **25.10.2023**
(21) Application number: 20941518.1
(22) Date of filing: 15.12.2020
(51) Int. Cl.: E04H 3/08, E04H 1/12, B01L 1/00

(54) **QUICK-BUILDING-TYPE TEST LABORATORY FOR MEDICAL TEST**
SCHNELL AUFBAUBARES TESTLABOR FÜR MEDIZINISCHE TESTS
LABORATOIRE DE TEST DE TYPE À CONSTRUCTION RAPIDE POUR TEST MÉDICAL

(30) Priority: 02.11.2020 CN 202022482114 U
(43) Date of publication of application: 22.06.2022
(73) Proprietor: BGI Genomics Co., Ltd., Yantian District Shenzhen, Guangdong Province 518083 (CN); Bgi Health (HK) Company Limited, Hong Kong (CN)
(72) Inventor: YIN, Ye, Shenzhen, Guangdong 518083 (CN); TANG, Meifang, Shenzhen, Guangdong 518083 (CN); ZENG, Hao, Shenzhen, Guangdong 518083 (CN); HUANG, Tianxun, Shenzhen, Guangdong 518083 (CN); SUO, Man, Shenzhen, Guangdong 518083 (CN); ZHANG, Dong, Shenzhen, Guangdong 518083 (CN); LIU, Xing, Shenzhen, Guangdong 518083 (CN); CHEN, Wurong, Shenzhen, Guangdong 518083 (CN); LI, Ning, Shenzhen, Guangdong 518083 (CN); LI, Wenqi, Shenzhen, Guangdong 518083 (CN); CAO, Sujie, Shenzhen, Guangdong 518083 (CN)
(74) Representative: DREISS Patentanwälte PartG mbB
(86) International application number: PCT/CN2020/136382
(87) International publication number: WO 2022/088421

(56) References cited:
- CN-A- 107 220 885
- CN-A- 111 484 929
- CN-A- 111 484 929
- CN-A- 111 502 350
- CN-A- 111 806 328
- CN-A- 111 826 281
- CN-U- 212 130 074
- DE-U1-202020 002 632
- US-A1- 2019 108 907

## Description

### FIELD

The present disclosure relates to the field of medical testing, particularly, to the field of clinical medical testing and public health testing, and more specifically, to a fast-built testing laboratory for medical testing, which is suitable for microbes, biochemical immunology, molecular diagnostics, and other applications of technical routes that have certain cleanliness requirements for the laboratory environment.

### BACKGROUND

Since the outbreak of the Corona Virus Disease 2019 (Covid-19) pandemic in early 2020, virus testing laboratories (or biosafety laboratories) for virus testing and research have also experienced serious shortages in addition to the huge demand for epidemic prevention hospitals, shelter hospitals, and isolation places. Once a sudden infectious disease epidemic or related event occurs, for example, when a new Covid-19 epidemic occurs, medical staffs need to immediately reach the scene to collect relevant samples, which may include humans, animals, environment, etc., and then transfer the relevant samples to a biosafety protection laboratory (testing laboratory), in which the potential biological hazards (pathogens, toxins, etc.) are detected and identified, so as to provide rapid laboratory diagnosis basis for the anti-epidemic front line, thereby achieving rapid response and timely providing decision-making basis for epidemic prevention and control.

Currently, it is particularly important to develop the capability of constructing large-scale and high-throughput temporary public health or clinical medical testing laboratories in a short period of time. Most of the conventional testing laboratories adopt prefabricated building structures, and a negative pressure system therein is required to treat the polluted air, which in turn leads to high implementation costs, long construction periods, difficulty in dismantling, as well as poor systematization and standardization of manufacturing and operation methods. In addition, the conventional testing laboratories are impossible to be packed and stored in advance, and a selection of a site thereof is also difficult. Meanwhile, when rebuilding a set of new testing products in different places, a series of measures must be taken to ensure a reliable testing quality. When the public health events and medical testing requirements erupt on a large scale, the existing conventional virus testing laboratories or biosafety laboratories obviously cannot meet the flexible and timely construction requirements. CN 111502350 A discloses a fast-built testing laboratory for medical testing according to the preamble of claim 1. Said laboratory is a pneumatic membrane structure virus detection lab including multiple pneumatic membrane structures; each pneumatic membrane structure can form a main space unit used as a main functional area and one or more sub space units used as auxiliary functional areas after being inflated; the main space units communicate with each other through the sub space units. CN 111484929 A discloses a movable pathogen nucleic acid testing laboratory system supporting rapid deployment. Three main steps of reagent storage and preparation, sample preparation, amplification and product analysis involved in a nucleic acid testing process are independently arranged in three movable structures respectively.

In addition, the conventional testing laboratory generally has a constant testing throughput, requires a long time for transformation, and cannot be flexibly and temporarily adjusted in response to different testing throughput requirements.

### SUMMARY

The present disclosure aims to solve one of the above-mentioned technical problems in the related art at least to a certain extent. For this purpose, the present disclosure provides a fast-built testing laboratory for medical testing, which can be rapidly constructed in an epidemic area and can also be rapidly adjusted according to a testing throughput, which is beneficial to the prevention and control of the epidemic. The fast-built testing laboratory of the invention is defined by the independent claim 1. Further advantageous embodiments are defined in the dependent claims thereon.

The fast-built testing laboratory for medical testing according to embodiments of the present disclosure includes a plurality of cabins, said cabins in the plurality of cabins being fast-built cabins and the plurality of cabins forming one or more testing chains. Each of the one or more testing chains includes: a sample unpacking zone; a sampling zone; a sample extraction zone; and a nucleic acid amplification and testing zone. For each of the one or more testing chains, extraction reagents required by the sample extraction zone and amplification reagents required by the nucleic acid amplification and testing zone are provided by a reagent preparation zone. A cabin quantity of the plurality of cabins is adjustable to be adapted to a testing throughput of the fast-built testing laboratory.

The fast-built testing laboratory for medical testing according to the embodiments of the present disclosure can be quickly built at a target location, and thus the testing laboratory can be put into use as soon as possible, which is conducive to the prevention and control of clinical or public health epidemics. In addition, the testing throughput of the fast-built testing laboratory can be flexibly changed by changing the number of cabins or adjusting a distribution of the respective zones in the cabins.

According to some embodiments of the present disclosure, the cabin quantity of the plurality of cabins is positively correlated with the testing throughput of the fast-built testing laboratory.

According to the present invention, the fast-built testing laboratory further includes a laboratory management system configured to manage sample data in the plurality of cabins.

According to some embodiments of the present disclosure, the plurality of cabins includes an unpacking cabin, a sampling cabin, an extraction cabin, an amplification cabin, and a reagent preparation cabin; and the sample unpacking zone is provided in the unpacking cabin, the sampling zone is provided in the sampling cabin, the sample extraction zone is provided in the extraction cabin, the nucleic acid amplification and testing zone is provided in the amplification cabin, and the reagent preparation zone is provided in the reagent preparation cabin.

Optionally, the plurality of cabins includes an integrated unpacking and sampling cabin; and the sample unpacking zone and/or the sampling zone is provided in the integrated unpacking and sampling cabin, and the integrated unpacking and sampling cabin is configured to supplement the unpacking cabin and/or the sampling cabin in quantity.

Further, the integrated unpacking and sampling cabin selectively serves as a sample unpacking zone and/or a sampling zone depending on a sample unpacking progress and a sampling progress as recorded in the laboratory management system.

According to some embodiments of the present disclosure, the cabin quantity of the plurality of cabins is sixteen, and the sixteen cabins include: a first unpacking cabin, a second unpacking cabin, a third unpacking cabin, a fourth unpacking cabin, a first sampling cabin, a second sampling cabin, a third sampling cabin, a forth sampling cabin, a first extraction cabin, a second extraction cabin, a third extraction cabin, a fourth extraction cabin, a first amplification cabin, a second amplification cabin, a reagent preparation cabin, and the integrated unpacking and sampling cabin; the sixteen cabins form at least four testing chains: a first testing chain including the first unpacking cabin, the first sampling cabin, the first extraction cabin, and the first amplification cabin; a second testing chain including the second unpacking cabin, the second sampling cabin, the second extraction cabin, and the first amplification cabin; a third testing chain including the third unpacking cabin, the third sampling cabin, the third extraction cabin, the second amplification cabin; and a fourth testing chain including the fourth unpacking cabin, the fourth sampling cabin, the fourth extraction cabin, and the second amplification cabin; the reagent preparation cabin is configured to provide extraction reagents for the first to fourth extraction cabins and provide amplification reagents for the first nucleic acid amplification and testing zone and the second nucleic acid amplification and testing zone; and the integrated unpacking and sampling cabin is configured to supplement the first to fourth unpacking cabins and/or the first to fourth sampling cabins in quantity.

According to some embodiments of the present disclosure, a sample obtained in the sample unpacking zone is marked with a sample code, and the laboratory management system is configured to allow input of information of the sample only when the sample code is verified.

According to some embodiments of the present disclosure, the laboratory management system is a localized laboratory management system supporting a local working mode, or a cloud platform laboratory management system supporting the local working mode and a remote collaborative working mode.

According to the present invention, the laboratory management system includes: a sample center, an experimental center, an analysis center, a report center, and a statistics center. In the sample center, samples are received, registered, and added with internal tracking numbers to form a package reception registration form. The experimental center includes the one or more testing chains, and in the experimental center, sample unpacking, sampling, sample extraction, and amplification are performed and data information generated in each process is recorded. The analysis center is configured to at least analyze data in the nucleic acid amplification and testing zone. The report center is configured to generate a report of a testing result of the nucleic acid amplification and testing zone. The report center is configured to generate a report of a testing result of the nucleic acid amplification and testing zone. The statistics center is configured to statistical obtain sample testing progresses and sample testing results.

According to some embodiments of the present disclosure, the sample unpacking zone and the sampling zone are located in different cabins or in a same cabin; the sampling zone and the sample extraction zone are located in different cabins or in a same cabin; the sample extraction zone and the reagent preparation zone are located in different cabins or in a same cabin; and the nucleic acid amplification and testing zone is located in a different cabin from any other zones.

According to some embodiments of the present disclosure, among the plurality of cabins, a number of cabins corresponding zones requiring longer operation time is greater than or equal to a number of cabins corresponding zones requiring shorter operation time.

According to some embodiments of the present disclosure, the plurality of cabins is constructed as inflatable film structures, tents, containers, or combinations thereof.

According to some embodiments of the present disclosure, two adjacent cabins of the plurality of cabins are configured to transfer materials there between through a transfer unit.

According to some embodiments of the present disclosure, the fast-built testing laboratory further includes a hazardous waste treatment system configured to treat medical waste generated in the plurality of cabins into non-hazardous waste.

According to some embodiments of the present disclosure, each of the plurality of cabins is a movable and fast-removable cabin.

According to some embodiments of the present disclosure, the fast-built testing laboratory includes: at least one first throughput unit each including three cabins of the plurality of cabins, the three cabins forming at least one of the one or more testing chains; and/or at least one second throughput unit each including four cabins of the plurality of cabins, the four cabins forming at least one of the one or more testing chains; and/or at least one third throughput unit each including five cabins of the plurality of cabins, the five cabins forming at least one of the one or more testing chains; and/or at least one fourth throughput unit each including eight cabins of the plurality of cabins, the eight cabins forming at least one of the one or more testing chains.

Optionally, the fast-built testing laboratory is provided with different testing throughputs by combining any one or more of the at least one first throughput unit, the at least one second throughput unit, the at least one third throughput unit, or the at least one fourth throughput unit.

Optionally, the fast-built testing laboratory includes one or more first throughput units, one or more second throughput units, one or more third throughput units, and one or more fourth throughput units.

According to some embodiments of the present disclosure, the three cabins in each of the at least one first throughput unit include a first mixing cabin, a second mixing cabin, and an amplification cabin; and the sample unpacking zone and the sampling zone are provided in the first mixing cabin, the sample extraction zone and the reagent preparation zone are provided in the second mixing cabin, and the nucleic acid amplification and testing zone are provided in the amplification cabin.

According to some embodiments of the present disclosure, the four cabins in each of the at least one second throughput unit include a first mixing cabin, a second mixing cabin, a third mixing cabin, and an amplification cabin; and the sample unpacking zone and the sampling zone are provided in the first mixing cabin, the sampling zone and the sample extraction zone are provided in the second mixing cabin, the sample extraction zone and the reagent preparation zone are provided in the third mixing cabin, and the nucleic acid amplification and testing zone is provided in the amplification cabin.

According to some embodiments of the present disclosure, the five cabins in each of the at least one third throughput unit include an unpacking cabin, a sampling cabin, an extraction cabin, an amplification cabin, and a mixing cabin; and the sample unpacking zone is provided in the unpacking cabin, the sampling zone is provided in the sampling cabin, the sample extraction zone is provided in the extraction cabin, the nucleic acid amplification and testing zone is provided in the amplification cabin, and the sample extraction zone and the reagent preparation zone are provided in the mixing cabin.

Optionally, the five cabins in each of the at least one third throughput unit include an unpacking cabin, a sampling cabin, an extraction cabin, an amplification cabin, and a mixing cabin; and the sample unpacking zone is provided in the unpacking cabin, the sampling zone is provided in the sampling cabin, the sample extraction zone is provided in the extraction cabin, the nucleic acid amplification and testing zone is provided in the amplification cabin, and the sample extraction zone and the reagent preparation zone are provided in the mixing cabin.

According to some embodiments of the present disclosure, the eight cabins in each of the at least one fourth throughput unit include a first unpacking cabin, a second unpacking cabin, a first sampling cabin, a second sampling cabin, a first extraction cabin, a second extraction cabin, an amplification cabin, and a reagent preparation cabin; and the sample unpacking zone is provided in the first unpacking cabin and the second unpacking cabin, the sampling zone is provided in the first sampling cabin and the second sampling cabin, the sample extraction zone is provided in the first extraction cabin and the second extraction cabin, the nucleic acid amplification and testing zone is provided in the amplification cabin, and the reagent preparation zone is provided in the reagent preparation cabin.

Optionally, the eight cabins in each of the at least one fourth throughput unit include an unpacking cabin, a first sampling cabin, a second sampling cabin, a first extraction cabin, a second extraction cabin, a first amplification cabin, a second amplification cabin, and a mixing cabin; and the sample unpacking zone is provided in the unpacking cabin, the sampling zone is provided in the first sampling cabin and the second sampling cabin, the sample extraction zone is provided in the first extraction cabin and the second extraction cabin, the nucleic acid amplification and testing zone is provided in the first amplification cabin and the second amplification cabin, and the sample extraction zone and the reagent preparation zone are provided in the mixing cabin.

The additional aspects and advantages of the present disclosure will be partly given in the following description, and part of them will become obvious from the following description, or can be understood through the practice of the present disclosure.

### BRIEF DESCRIPTION OF DRAWINGS

FIG. 1 is a schematic diagram of a first throughput unit according to an embodiment;
FIG. 2 is a schematic diagram of a second throughput unit according to an embodiment;
FIG. 3 is a schematic diagram of a third throughput unit according to an embodiment;
FIG. 4 is a schematic diagram of a third throughput unit according to another embodiment;
FIG. 5 is a schematic diagram of a fourth throughput unit according to an embodiment;
FIG. 6 is a schematic diagram of a fourth throughput unit according to another embodiment;
FIG. 7 is a schematic diagram of a testing throughput of 100,000 according to an embodiment;
FIG. 8 is a schematic diagram of a testing throughput of 100,000 according to another embodiment;
FIG. 9 is a schematic diagram of a testing throughput of 300,000 according to an embodiment;
FIG. 10 is a schematic diagram of a testing chain with a testing throughput of 300,000 according to an embodiment;
FIG. 11 is a schematic diagram of quality control standards;
FIG. 12 is a schematic diagram of result evaluation criteria; and
FIG. 13 is a schematic diagram of an entire process of information flow transmission with samples as a main line.

### Reference numerals:

1: sample unpacking zone; 2: sampling zone; 3: sample extraction zone; 4: nucleic acid amplification and testing zone; 5: reagent preparation zone; 11: unpacking cabin; 12: sampling cabin; 13: extraction cabin; 14: amplification cabin; 15: reagent preparation cabin; 16: mixing cabin; 17: integrated unpacking and sampling cabin.

### DESCRIPTION OF EMBODIMENTS

Embodiments of the present disclosure are described in detail below. Examples of the embodiments are illustrated in the accompanying drawings, throughout which the same or similar reference numerals represent the same or similar elements or elements with the same or similar functions. The embodiments described below with reference to the drawings are illustrative, and are intended to explain rather than limit the present disclosure.

In the present disclosure, it should be understood that terms "length", "width", "upper", "lower", "front", "rear", "left", "right", "top", "bottom", "inner", "outer", etc. indicate orientation or positional relationships based on the orientation or positional relationships shown in the drawings, and are only for the convenience of description and simplifying the description, rather than indicating or implying that a designated device or component must have a specific orientation or must be constructed and operated in a specific orientation. In this regard, these terms cannot be understood as limitations to the present disclosure.

In addition, terms "first" and "second" are merely used for descriptive purposes, and cannot be understood as indicating or implying relative importance or implicitly indicating the number of indicated technical features. Thus, the features defined with "first" and "second" may explicitly or implicitly include one or more of these features. In the present disclosure, "plurality of" means at least two, for example, two, three, etc., unless otherwise specifically defined.

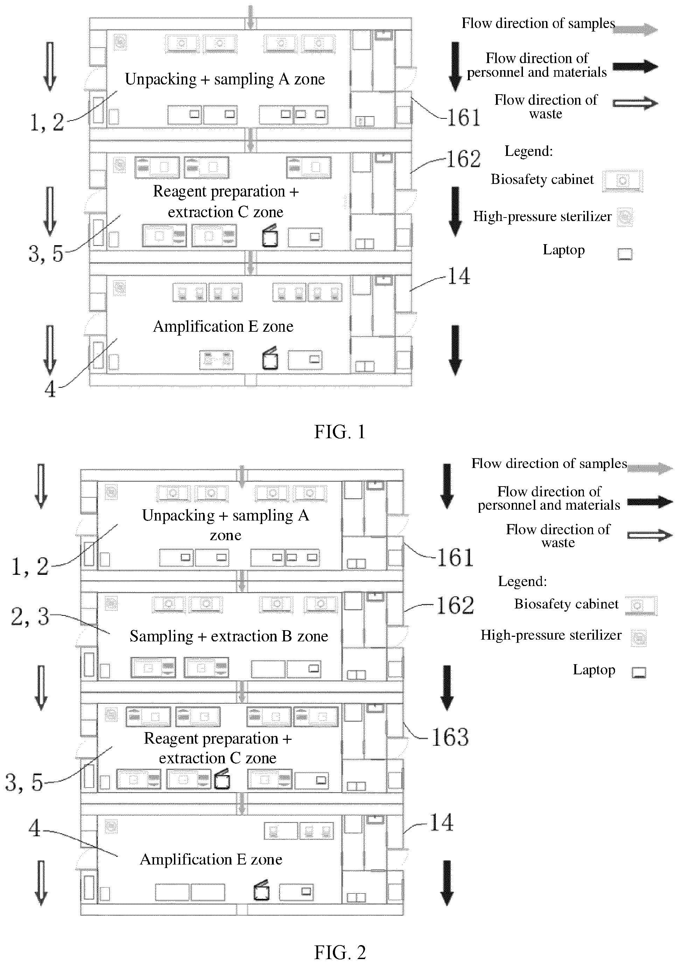

A fast-built testing laboratory for medical testing according to the embodiments of the present disclosure is described in detail below with reference to FIG. 1 to FIG. 13.

Referring to FIG. 1 to FIG. 10, the fast-built testing laboratory for medical testing according to the embodiments of the present disclosure can include a plurality of cabins. The cabins are fast-built cabins. The plurality of cabins forms one or more testing chains. Each testing chain includes: a sample unpacking zone 1, a sampling zone 2, a sample extraction zone 3, a nucleic acid amplification and testing zone 4. In the sample unpacking zone 1, a sample package can be unpacked and inactivated. In the sampling zone 2, the sample can be transferred to a plate. In the sample extraction zone 3, virus, nucleic acid and other test substances in the sample can be extracted. In the nucleic acid amplification and testing zone 4, the extracted virus, nucleic acid and other test substances can be amplified.

For each testing chain, extraction reagents required by the sample extraction zone 3 and amplification reagents required by the nucleic acid amplification and testing zone 4 are provided by a reagent preparation zone 5. The extraction reagents are used to extract the virus, nucleic acid and other test substances from the sample, and the amplification reagents are used to amplify the extracted virus, nucleic acid and other test substances. The reagent preparation zone 5 serves as a zone for providing reagents and is arranged inside the fast-built testing laboratory, and the reagent preparation zone 5 may be close to the sample extraction zone 3 and the nucleic acid amplification and testing zone 4, which facilitates the rapid transfer of the extraction reagents and the amplification reagents to the corresponding sample extraction zone 3 and nucleic acid amplification and testing zone 4. In addition, the reagent preparation zone 5 provides the extraction reagents and the amplification reagents, which can reduce the difference in reagents used in the respective cabins and facilitate source tracing of the reagents.

In some alternative embodiments, according to the requirements on laboratory testing quality and management system, a dedicated cabin can also be provided to complete the verification, re-check, and retest operation of the sample.

Samples and information of each testing chain are only transferred within the testing chain by an exclusive transfer personnel, thereby avoiding chaos that may occur in a case of a large sample volume.

Instruments, devices, etc. required for the respective steps can be placed in the sample unpacking zone 1, the sampling zone 2, the sample extraction zone 3, the nucleic acid amplification and testing zone 4, and the reagent preparation zone 5. As an optional embodiment, configurations of the instruments and devices in the zones are listed in Table 1:

[Table 1] Device configurations of sample unpacking zone 1, the sampling zone 2, the sample extraction zone 3, the nucleic acid amplification and testing zone 4, the reagent preparation zone 5

| Spatial function | Name of key devices | Number of devices for verification scheme | Spatial function | Name of key devices | Number of devices for verificatio n scheme |
|---|---|---|---|---|---|
| Sample unpacking zone 1 | Biosafety cabinet | 4 | Sampling zone 2 | Biosafety cabinet | 6 |
| | General experiment table | 4 | | General experiment table | 2 |
| | High-pressure sterilizer | 1 | | High-pressure sterilizer | 1 |
| | Barcode scanner | 4 | | Printer | 1 |
| | Printer | 1 | | Barcode printer | 1 |
| | Barcode printer | 1 | | Barcode scanner | 1 |
| | Laptop | 5 | | Laptop | 1 |
| Sample extraction zone 3 | MGISP-960 (including computer) | 7 | Reagent preparation zone 5 | MGISP-960 (including computer) | 6 |
| | Equipment table | 7 | | Equipment table | 6 |
| | General experiment table | 1 | | General experiment table | 1 |
| | Vertical refrigerator | 1 | | Vertical refrigerator | 1 |
| | High-pressure sterilizer | 1 | | High-pressure sterilizer | 1 |
| | Barcode scanner | 1 | | Barcode scanner | 1 |
| | Laptop | 1 | | Laptop | 1 |
| Nucleic acid amplification and testing zone 4 | PCR machine (including computer) | 34 | | | |
| | Three-tier shelf | 6 | | | |
| | General experiment table | 3 | | | |
| | Vertical refrigerator | 1 | | | |
| | Laptop | 1 | | | |

In some other embodiments, in order to maximize the use of the space in the cabins and enhance the testing throughput, the high-pressure sterilizer may be not provided inside the cabins, but provided collectively at a special position and managed by a dedicated person.

The cabin quantity of the plurality of cabins is adjustable to be adapted to a testing throughput of the fast-built testing laboratory. For example, the testing throughput of the fast-built testing laboratory is designed based on the severity of the Covid-19 epidemic, and the number of cabins is adjusted according to the testing throughput of the fast-built testing laboratory, allowing the number of cabins to match the testing throughput of the fast-built testing laboratory. In other words, according to the requirements on the testing throughput of a large-scale laboratory, the above-mentioned sample unpacking zone 1, sampling zone 2, sample extraction zone 3, nucleic acid amplification and testing zone 4, and reagent preparation zone 5 are arbitrarily combined by taking the cabin as unit, and proportions of the respective functional cabins are flexibly adjusted, to reach the optimal throughput and productivity of the laboratory.

The outbreak of epidemic is very sudden. Taking the sudden Covid-19 outbreak as an example, it is difficult to predict the city where the epidemic will break out. Once the epidemic breaks out, it is necessary to quickly establish nucleic acid testing laboratories at the outbreak site. The fast-built testing laboratory for medical testing according to the embodiments of the present disclosure can be used as a temporary emergency facility, which can be rapidly and flexibly constructed at different target locations (where the epidemic occurs), and a P2 or P2+ level laboratory can be built in a few days, which can save the time of constructing a testing laboratory and enable the testing laboratory to be putted into use as soon as possible. In this way, the time for testing and identifying relevant samples can be shorten, which is conducive to the prevention and control of clinical or public health epidemics. In addition, the number of cabins or adjusting a distribution of respective functional zones in the cabins (i.e., the sample unpacking zone 1, the sampling zone 2, the sample extraction zone 3, the nucleic acid amplification and testing zone 4, and the reagent preparation zone 5) can be changed to adapt to the testing processes of different technical routes and flexibly change the testing throughput of the fast-built testing laboratory. Furthermore, the fast-built testing laboratory also has extremely high flexibility and strong environmental adaptability. It is not only suitable for various indoor places, but also resistant to all kinds of outdoor wind, frost, rain and snow weathers, and is of great significance for the sudden public health and clinical testing needs, epidemic prevention and control, large population molecular testing or nucleic acid testing, etc.

The fast-built testing laboratory has good airtightness, and is equipped with a high-efficiency fresh air filtration system. The air passing through an air inlet and an air outlet is subjected to a high-efficiency filtration, and the air is discharged after harmless disposal, thereby ensuring the cleanliness of the air inside and outside the cabin. In this way, it is guaranteed that the temperature and humidity in the cabins meet the requirements.

A sample management zone, a warehouse and material transfer zone, a waste treatment zone, an IT storage zone, a data analysis office zone and other auxiliary zones may be provided outside the sample unpacking zone 1, the sampling zone 2, the sample extraction zone 3, the nucleic acid amplification and testing zone 4, and the reagent preparation zone 5, so as to support smooth progresses of works in the sample unpacking zone 1, the sampling zone 2, the sample extraction zone 3, the nucleic acid amplification and testing zone 4, and the reagent preparation zone 5 from the outside. The auxiliary zones may be provided in some cabins, or in additional rooms outside the cabins.

In some embodiment of the present disclosure, the number of the plurality of cabins is positively correlated with the testing throughput of the fast-built testing laboratory. In other words, the testing throughput of the fast-built testing laboratory can be enhanced by increasing the number of cabins, so as to accelerate the testing.

The fast-built testing laboratory further includes a laboratory management system, and the laboratory management system is configured to manage sample data in the plurality of cabins.

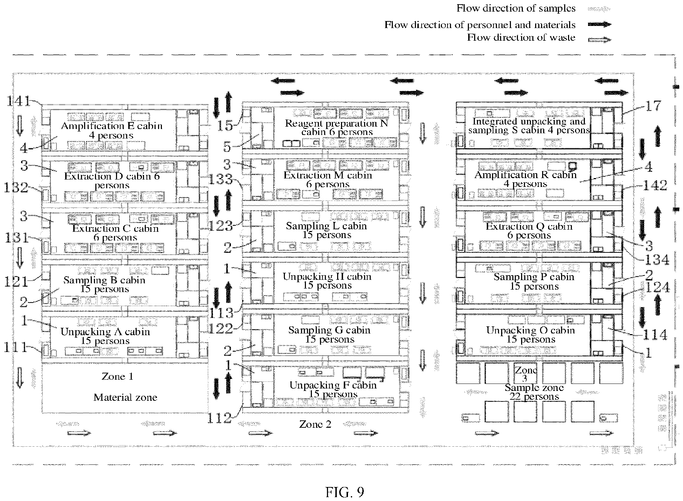

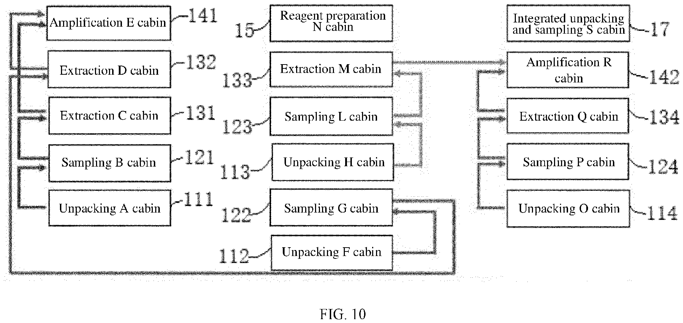

According to the present invention, the plurality of cabins includes: an unpacking cabin 11, a sampling cabin 12, an extraction cabin 13, an amplification cabin 14, and a reagent preparation cabin 15. The sample unpacking zone 1 is provided in the unpacking cabin 11, the sampling zone 2 is provided in the sampling cabin 12, the sample extraction zone 3 is provided in the extraction cabin 13, the nucleic acid amplification and testing zone 4 is provided in the amplification cabin 14, and the reagent preparation zone 5 is provided in the reagent preparation cabin 15. In the embodiment as illustrated in FIG. 9 and FIG. 10, in view of the correspondence relationship between the cabins and the functional zones, each cabin has one single zone. That is, only the sample unpacking zone 1 is provided in the unpacking cabin, only the sampling zone 2 is provided in the sampling cabin, only the sample extraction zone 3 is provided in the extraction cabin, only the nucleic acid amplification and testing zone 4 is provided in the amplification cabin, and only the reagent preparation zone 5 is provided in the reagent preparation cabin.

Of course, it should be noted that the cabins may be provided with other auxiliary zones inside in addition to the functional zones, for example, a disinfection zone, a waste disposal zone, etc., which will not be individually listed herein. "Only the sample unpacking zone 1 is provided in the unpacking cabin" should be construed as that only one functional zone (i.e., the sample unpacking zone 1) is provided in the unpacking cabin, and other functional zones such as the sampling zone 2, the sample extraction zone 3, the nucleic acid amplification and testing zone 4, and the reagent preparation zone 5 are not provided in the unpacking cabin. Other similar expressions shall be construed in the same way, and are not individually explained herein.

Optionally, as illustrated in FIG. 9 and FIG. 10, the fast-built testing laboratory further includes an integrated unpacking and sampling cabin 17; the sample unpacking zone 1 and/or the sampling zone 2 are provided in the integrated unpacking and sampling cabin 17; the integrated unpacking and sampling cabin 17 is configured to supplement the unpacking cabin and/or the sampling cabin in quantity. In other words, according to the requirements on the testing throughput of the fast-built testing laboratory, the integrated unpacking and sampling cabin 17 can optionally serve as the unpacking cabin and/or the sampling cabin, so as to accelerate the unpacking and/or sampling.

The testing laboratory illustrated in FIG. 9 and FIG. 10 includes sixteen cabins in total. The sixteen cabins include: first to fourth unpacking cabins, first to fourth sampling cabins, first to fourth extraction cabins, first to second amplification cabins, a reagent preparation cabin, and an integrated unpacking and sampling cabin. The sixteen cabins form at least four testing chains, i.e., a testing chain 1 including an unpacking A cabin 111, a sampling B cabin 121, an extraction C cabin 131, and an amplification E cabin 141; a testing chain 2 including an unpacking F cabin 112, a sampling G cabin 122, an extraction D cabin 132, and an amplification E cabin 141; a testing chain 3 including an unpacking H cabin 113, a sampling L cabin 123, an extraction M cabin 133, and an amplification R cabin 142; and a testing chain 4 including an unpacking O cabin 114, a sampling P cabin 124, an extraction Q cabin 134, and an amplification R cabin 142.

A reagent preparation N cabin 15 is configured to provide extraction reagents for the extraction C cabin 131, the extraction D cabin 132, the extraction M cabin 133, and the extraction Q cabin 134, and provide amplification reagents for the amplification E cabin 141 and the amplification R cabin 142.

An integrated unpacking and sampling S cabin 17 is configured to supplement the first to fourth unpacking cabins 111 to 114 and/or the first to fourth sampling cabins 121 to 124. In other words, the sample unpacking zone 1 and/or the sampling zone 2 may be optionally provided in the integrated unpacking and sampling S cabin 17.

In addition, a specific zone, responsible for sampling operations of all samples that are required to be re-extracted and re-amplified, may be provided in the sampling G cabin 122.

Further, the integrated unpacking and sampling cabin 17 optionally serves as the sample unpacking zone 1 and/or the sampling zone 2 based on a sample unpacking progress and a sampling progress that are recorded in the laboratory management system. That is, by querying the sample unpacking progress and the sampling progress recorded in the laboratory management system, the speeds of unpacking and sampling can be known timely. When the speed of sample unpacking is relatively slow, the integrated unpacking and sampling cabin 17 can serve as the sample unpacking zone 1. When the speed of sampling is relatively slow, the integrated unpacking and sampling cabin 17 can serve as the sampling zone 2. When the speed of sample unpacking is basically the same as the speed of sampling, a part of the integrated unpacking and sampling cabin 17 can serve as the sample unpacking zone 1, and the other part of the integrated unpacking and sampling cabin 17 can serve as the sampling zone 2.

In some embodiment of the present disclosure, a sample obtained in the sample unpacking zone 1 is marked with a sample code, and the laboratory management system is configured to allow an input of sample information only when the sample code is verified.

Specifically, in order to ensure the accuracy and safety of transmission of a laboratory testing information flow, settings can be made when the sample code is inputted. Further, in order to protect personal information from being leaked, the sample code is used as a unique identification in the entire testing process, without leaking any personal information.

In some embodiment of the present disclosure, the sample code is in a form of a 9-digit code + a 1-digit check code, the first digit is an English letter, which represents a name of an organization, and the following 9 digits vary with different sample tubes. The calculation of the check code is explained by taking the code "B43819206" as an example. "B" represents the BGI GENOMICS CO., LTD., 10×2(B=2)+9×4+8×3+7×8+6×1+5×9+4×2+3×0+2×6=207, then 207 is divided by 11 to obtain a remainder, 9, a check number=11-9(remainder)= 2, and thus the complete code of the sample is B438192062. If the obtained check number is "10", "A" is used to represent the check number; and if the remainder after the division by 11 is 0, then the check number is 0. The laboratory management system will check the uniqueness of the sample code, and only the sample information meeting the rules can be inputted, thereby preventing the unknown and abnormal source samples from entering the testing process.

Subsequently, the code information transmission of a single sample is switched to 96-well plate information transmission. That is, every 96 reactions are arranged in one task list, and the 96 reactions include 92 test samples, 2 negative controls, 1 positive control, and 1 blank control. The task list follows such a coding rule: place name + date + a name of an unpacking cabin + a 3-digit serial number name, and if digits of the serial number are less than 3 digits, 0 can be made up, such as ZS-0901-A-001. When multiple samples are mixed to be tested, the samples need to be mixed on a pooling plate. The Pooling plate follows such a coding rule: place name + date + a name of an extraction cabin + P plus 3 digits serial number name, and if digits of the serial number are less than 3 digits, 0 can be made up, for example, ZS-0901-C-P001.

In some embodiment of the present disclosure, the laboratory management system is a localized laboratory management system or a cloud platform laboratory management system. The localized laboratory management system supports a local working mode, can realize a physical isolation, and can customize data access authority and information security protocol. The cloud platform laboratory management system supports a local working mode and a remote collaborative working mode. The cloud platform laboratory management system needs to use a cloud platform to realize data information management. With the increase of testing volume, when the data volume in the middle and late stages rises rapidly, a volume expansion load plan for the cloud platform is adopted to ensure a rapid transfer of data and information. In addition, cloud-native full-link encryption is used to protect data to ensure data security on the cloud. Further, the cloud platform laboratory management system can also use resources flexibly. Furthermore, an automatic data backup processing can be performed on the cloud, which further guarantees data security.

That is, the laboratory management system of the fast-built testing laboratory can be deployed offline, supporting offline production and use, and it can manage the whole process information of the experiments, ensure smooth information transfer and data security. The laboratory management system can also be used on the cloud, so as to facilitate the unified management and data exchange of the fast-built testing laboratories in various regions.

When data of the fast-built testing laboratory is delivered, the system supports a manual delivery mode of locally exporting an Excel file, and also supports an API delivery mode, which can be connected to other local laboratory management systems to automatically transmit data.

According to the present invention, the laboratory management system includes a sample center, an experimental center, an analysis center, a report center, and a statistics center. In the sample center, a sample is received, registered, and added with an internal tracking number to form a package reception registration form. The experimental center includes the testing chains, and in the experimental center, sample unpacking, sampling, sample extraction, and amplification are performed and data information generated in each process is recorded. The analysis center is configured to at least analyze data in the nucleic acid amplification and testing zone 4. The report center is configured to generate a report of a testing result of the nucleic acid amplification and testing zone 4. The statistics center is configured to statistically calculate sample testing progresses and sample testing results, and the statistical data can be retrieved from the statistics center every 2 hours to obtain data such as the number of samples received, the number of unpackings, the number of samples, the number of extractions, the number of amplifications, and the number of data analysis. The data can help the coordinator to pay close attention to the progress of the experiments at any time, and monitor the efficiency of the experiment of each cabin, ensuring that problems can be found and solved in time. If an abnormality occurs, the actual sample tube or task list can be found and reconfirmed by tracing the information source.

The transfer of data information among the sample unpacking zone 1, the sampling zone 2, the sample extraction zone 3, the nucleic acid amplification and testing zone 4, and the reagent preparation zone 5 can be achieved through the laboratory management system.

Data of all experimental processes is collectively processed in the laboratory management system, and experimenters record data, retrieve data, manage experimental progresses, and trace information in the laboratory management system. Each experimenter has a unique account. A person in charge of the laboratory assigns the accounts and sets user permissions. Personnel in different posts can only see relevant data information required by their posts, thereby preventing data from being tampered or leaked at will.

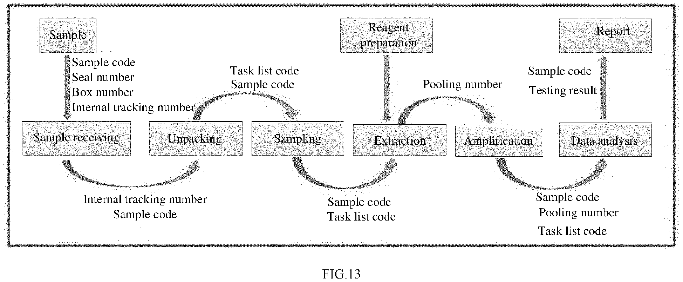

FIG. 13 illustrates a schematic diagram of an entire process of information flow transmission with samples as the main line. Samples are first received, registered and added with internal tracking numbers in the sample center. This step generates a package reception registration form. A sample delivery box number, a seal number, the internal tracking numbers, and the number of the samples are required to be filled into the registration form. The package, after being registered, will be transferred to the sample unpacking zone 1 for unpacking and scheduling, and meanwhile, the sample information is inputted in the laboratory management system. This step can check whether the number of samples with the corresponding internal tracking number is correct. After the scheduling, a task list is generated and a task list code is assigned to the task list. The task list has a check formula, in order to check whether the sample code conforms to the following rules: the sample code has 10 digits, starting with a capital letter B, and the last digit can be a number or capital letter. If the sample code fails to conform to the rules, it is necessary to check whether the inputted sample code is wrong. A paper sheet of the task list will be generated and transferred to a subsequent step. In the sampling step, a barcode of the task list code is required to be pasted on the paper sheet, to check whether the task list code is consistent with the sample tube code, thereby preventing code or position mistakes caused by errors in the scheduling. In the meantime, the real-time sampling registration form is registered to confirm whether the sampling has been completed. After the sampling is completed, the paper sheet and sample deep-well plates are transferred to the sample extraction zone 3. Regarding the samples for pool testing, 3 sample plates are pooled and mixed in the sample extraction zone 3, and then the mixed samples are extracted. In this step, the task list codes of 3 pooling plates are scanned and inputted in an extraction functional module of the system. The system generates post-pooling plate codes and matches information of the four plate codes, and the corresponding 3 paper sheets are bound and pasted with barcode of the post-pooling plate codes, extraction reagent batches, and QPCRmix reagent batches. After the extraction is completed, the extracted information is automatically online synchronized to the amplification step. The extracted nucleic acid is mixed with QPCRmix and transferred with the bound paper sheets to the nucleic acid amplification and testing zone 4. In the nucleic acid amplification and testing zone 4, a QPCR task schedule is required to be registered, and at the same time, it is checked whether the task list code is consistent with the online electronic task list code. After the amplification is completed, the analysis center will analyze the results and generate the test results of each sample. The result information will be stored in the online system. After the results are confirmed to be correct, the data coordinator can issue a sample destruction instruction. After receiving the destruction instruction, the sample center will package the samples to be destroyed and deliver them to the on-site team to complete the final destruction. The report center sends the sample results to the government.

In the entire process, each step can check whether the information generated in the previous step is accurate, so as to effectively find problems and promptly report the problems to a superior for solution. In every important step, such as sampling, extraction, or other error-prone steps, two people perform double-check to reduce errors caused by manual operations. In addition, paper sheets, such as a task list, a transfer and delivery form, a reagent preparation form, and a computer record form, are generated. On the one hand, the paper sheets facilitate the information check in the subsequent processes, and on the other hand, original records can be reserved to trace the source and correct the information errors that may be caused by mis-operation of the electronic record sheets. In addition, when the sample codes and plate codes are inputted into the online system, the field rules will also be checked. The codes failing to conform to the rules cannot be input into the system, thereby preventing result errors caused by code errors.

As illustrated in FIG. 1 to FIG. 10, in some embodiment of the present disclosure, the sample unpacking zone 1 and the sampling zone 2 are located in different cabins or in the same cabin; the sampling zone 2 and the sample extraction zone 3 are located in different cabins or in the same cabin; the sample extraction zone 3 and the reagent preparation zone 5 are located in different cabins or in the same cabin; and the nucleic acid amplification testing zone 4 is located in a cabin different from the other zones.

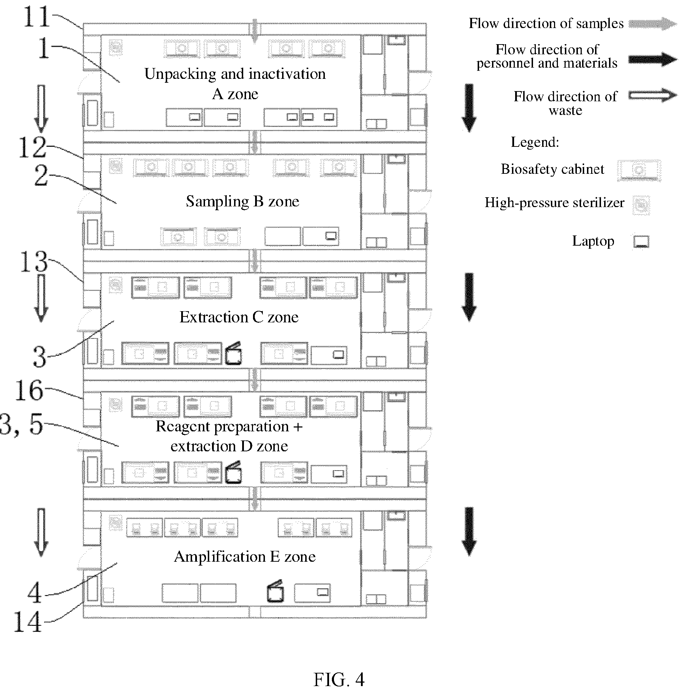

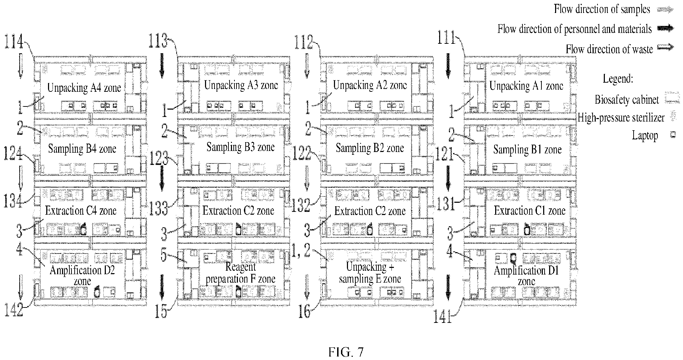

As illustrated in FIG. 1, the sample unpacking zone 1 and the sampling zone 2 are located in the same cabin (mixing cabin 161), the sample extraction zone 3 and reagent preparation zone 5 are located in the same cabin (mixing cabin 162). As illustrated in FIG. 2, the sample unpacking zone 1 and the sampling zone 2 are located in the same cabin (mixing cabin 161), the sampling zone 2 and the sample extraction zone 3 are located in the same cabin (mixing cabin 162), and the sample extraction zone 3 and the reagent preparation zone 5 are located in the same cabin (mixing cabin 163). As illustrated in FIG. 4, FIG. 6, and FIG. 8, the sample extraction zone 3 and reagent preparation zone 5 are located in the same cabin (mixing cabin 16). As illustrated in FIG. 7, the sample unpacking zone 1 and the sampling zone 2 are located in the same cabin (mixing cabin 16). In FIG. 1 to FIG. 9, one single functional zone is provided in each of the cabins except for the mixing cabin 16. For example, only the sample unpacking zone 1 is provided in the unpacking cabin 11, only the sampling zone 2 is provided in the sampling cabin 12, only the sample extraction zone 3 is provided in the extraction cabin 13, only the nucleic acid amplification and testing zone 4 is provided in the amplification cabin 14, and only the reagent preparation zone 5 is provided in the reagent preparation cabin 15.

As illustrated in FIG. 1 to FIG. 10, the nucleic acid amplification and testing zone 4 is located in a different cabin from the sample unpacking zone 1, the sampling zone 2, the sample extraction zone 3, and the reagent preparation zone 5. Since the nucleic acid amplification and testing zone 4 requires performing an exponential amplification on a product to be tested, if the product to be tested is a hazardous substance, it is likely to cause leakage and personnel infection accidents. Therefore, the nucleic acid amplification and testing zone 4 is provided in a separate cabin, which is conducive to the use safety of the fast-built testing laboratory.

In some embodiment of the present disclosure, a number of cabins corresponding to a zone for a step requiring longer time is greater than or equal to a number of cabins corresponding a zone for a step requiring shorter time. In other words, the zone for a step requiring longer time has a slower progress. By increasing the number of the cabins corresponding to the zone for a step requiring longer time, the efficiency of the step can be improved. For example, the speeds of unpacking and sampling are relatively slow, by increasing the number of sample unpacking zones 1 and the number of sampling zones 2, the unpacking and the sampling can be speeded up, so as to provide sufficient sample volume for the subsequent sampling and amplification, thereby ensuring the smooth testing process without interruption.

In some embodiment of the present disclosure, the plurality of cabins is constructed as inflatable film structures, tents, containers, or combinations thereof. The inflatable film structures and the tents can be folded for transportation, and after the testing is completed, they can be quickly dismantled and transported to other places for use. The containers can also be designed as foldable or drawing-type containers, which are convenient to be transported to other places after being dismantled. A single cabin has the advantages of moderate volume and easy transportation. The number of cabins can be changed arbitrarily according to the requirements, in order to realize the flexible arrangement of the testing laboratory. Undoubtedly, with the development of the technology, the cabin may also be developed into a fast-built cabin in other forms.

In some embodiment of the present disclosure, every two adjacent cabins of the plurality of cabins are adapted to transfer materials there between through a transfer unit. The delivery unit can be a delivery box. For example, the virus, nucleic acid and other test substances extracted in the sample extraction zone 3 can be delivered to the nucleic acid amplification and testing zone 4 via the transfer unit, without requiring the experimenter to enter the nucleic acid amplification and testing zone 4 from the sample extraction zone 3, thereby reducing the flow of personnel. Thus, the safety of the testing process can be improved and the time for personnel walking is saved, thereby improving the testing efficiency.

In some embodiment of the present disclosure, the fast-built testing laboratory further includes a hazardous waste treatment system. The hazardous waste treatment system is configured to treat medical waste generated in the cabins into non-hazardous waste, and then discharge the harmless solid waste, waste water, etc. out of the laboratory.

In some embodiment of the present disclosure, the cabins are movable and fast-removable cabins. In this way, when the epidemic in the target place is eliminated, the laboratory can be dismantled into the smallest cabins, and then the cabins can be transported to a new target site, which is conducive to the reuse of respective devices. After the laboratory is dismantled, the occupied space is released and thus can be re-used.

In some embodiments of the present disclosure, the fast-built testing laboratory include: at least one first throughput unit, each including three cabins, the three cabins forming at least one testing chain; and/or at least one second throughput unit, each including four cabins, the four cabins forming at least one testing chain; and/or at least one third throughput unit, each including five cabins, the five cabins forming at least one testing chain; and/or at least one fourth throughput unit, each including eight cabins, the eight cabins forming at least one testing chain.

In a specific embodiment, the first throughput unit can reach a testing throughput of 10,000 per day, the second throughput unit can reach a testing throughput of 20,000 per day, the third throughput unit can reach a testing throughput of 30,000 per day, and the fourth throughput unit can reach a testing throughput of 50,000 per day.

As illustrated in FIG. 1, the first throughput unit includes three cabins, i.e., a first mixing cabin 161, a second mixing cabin 162, and an amplification cabin 14. In view of a correspondence relationship between the cabins and the functional zones, the sample unpacking zone 1 and the sampling zone 2 are provided in the first mixing cabin 161; the sample extraction zone 3 and the reagent preparation zone 5 are provided in the second mixing cabin 162; and only the nucleic acid amplification and testing zone 4 is provided in the amplification cabin 14. These three cabins, i.e., the first mixing cabin 161, the second mixing cabin 162, and the amplification cabin 14 form one testing chain. A flow direction of samples, a flow direction of personnel and materials, and a flow direction of waste each can be: the first mixing cabin 161 → the second mixing cabin 162 → the amplification cabin 14.

As illustrated in FIG. 2, the second throughput unit includes four cabins: a first mixing cabin 161, a second mixing cabin 162, a third mixing cabin 163, and an amplification cabin 14. In view of a correspondence relationship between the cabins and the functional zones, the sample unpacking zone 1 and the sampling zone 2 are provided in the first mixing cabin 161, the sampling zone 2 and the sample extraction zone 3 are provided in the second mixing cabin 162, the sample extraction zone 3 and reagent preparation zone 5 are provided in the third mixing cabin 163, and only the nucleic acid amplification and testing zone 4 is provided in the amplification cabin 14. These four cabins, i.e., the first mixing cabin 161, the second mixing cabin 162, the third mixing cabin 163, and the amplification cabin 14 form at least one testing chain. A flow direction of samples, a flow direction of personnel and materials, and a flow direction of waste each can be as follow: the first mixing cabin 161 → the second mixing cabin 162 → the third mixing cabin 163 → the amplification cabin 14.

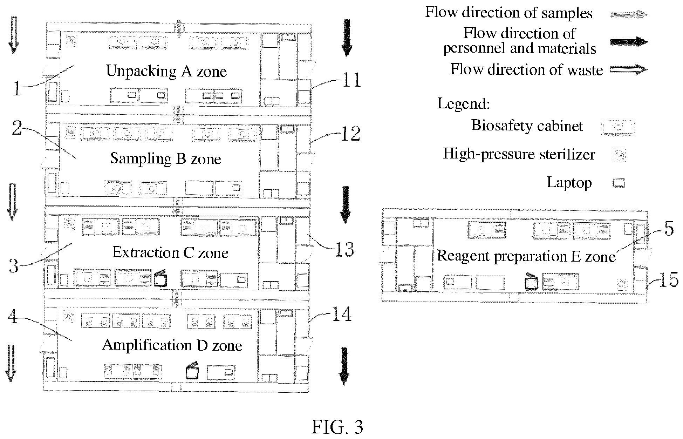

As illustrated in FIG. 3, the third throughput unit according to a first embodiment includes five cabins: an unpacking cabin 11, a sampling cabin 12, an extraction cabin 13, an amplification cabin 14, and a reagent preparation cabin 15. In view of a correspondence relationship between the cabins and the functional zones, only the sample unpacking zone 1 is provided in the unpacking cabin 11, only the sampling zone 2 is provided in the sampling cabin 12, only the sample extraction zone 3 is provided in the extraction cabin 13, only the nucleic acid amplification and testing zone 4 is provided in the amplification cabin 14, and only the reagent preparation zone 5 is provided in the reagent preparation cabin 15. A flow direction of samples, a flow direction of personnel and materials, and a flow direction of waste each can be as follow: the unpacking cabin 11 → the sampling cabin 12 → the extraction cabin 13 → the amplification cabin 14. These five cabins form one testing chain: the unpacking A cabin 11 - the sampling B cabin 12 - the extraction C cabin 13 - the amplification D cabin 14.

As illustrated in FIG. 4, the third throughput unit according to a second embodiment includes five cabins: an unpacking cabin 11, a sampling cabin 12, an extraction cabin 13, an amplification cabin 14, and a mixing cabin 16. In view of a correspondence relationship between the cabins and the functional zones, only the sample unpacking zone 1 is provided in the unpacking cabin 11, only the sampling zone 2 is provided in the sampling cabin 12, only the sample extraction zone 3 is provided in the extraction cabin 13, only the nucleic acid amplification and testing zone 4 is provided in the amplification cabin 14, and the sample extraction zone 3 and the reagent preparation zone 5 are provided in the mixing cabin 16. A flow direction of samples, a flow direction of personnel and materials, and a flow direction of waste each can be as follow: the unpacking cabin 11 → the sampling cabin 12 → the extraction cabin 13 → the mixing cabin 16 → the amplification cabin 14. These five cabins at least form one testing chain: the unpacking A cabin 11 - the sampling B cabin 12 - the extraction C cabin 13 - the amplification E cabin 14. In addition, some of the operations in the extraction C zone can be performed in the extraction D zone.

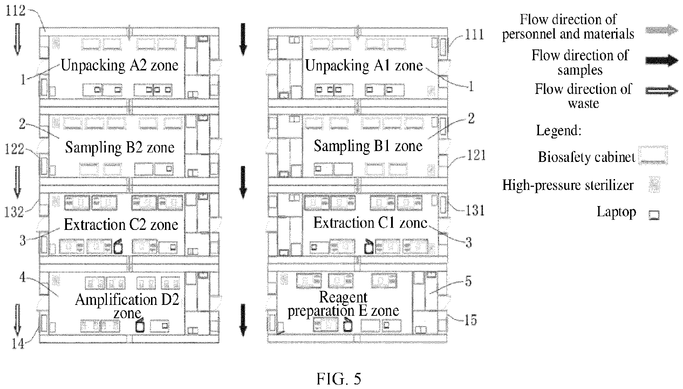

As illustrated in FIG. 5, the fourth throughput unit according to a first embodiment includes eight cabins: a first unpacking cabin 111, a second unpacking cabin 112, a first sampling cabin 121, a second sampling cabin 122, a first extraction cabin 131, a second extraction cabin 132, an amplification cabin 14, and a reagent preparation cabin 15. In view of a correspondence relationship between the cabins and the functional zones, only the sample unpacking zone 1 is provided in the first unpacking cabin 111 and the second unpacking cabin 112, only the sampling zone 2 is provided in the first sampling cabin 121 and the second sampling cabin 122, only the sample extraction zone 3 is provided in the first extraction cabin 131 and the second extraction cabin 132, only the nucleic acid amplification and testing zone 4 is provided in the amplification cabin 14, and only the reagent preparation zone 5 is provided in the reagent preparation cabin 15. These eight cabins form two testing chains, i.e., a testing chain 1 including the unpacking A1 cabin 111, the sampling B1 cabin 121, the extraction C1 cabin 131, and the amplification D2 cabin 14; and a testing chain 2 including the unpacking A2 cabin 112, the sampling B2 cabin 122, the extraction C2 cabin 132, and the amplification D2 cabin 14.

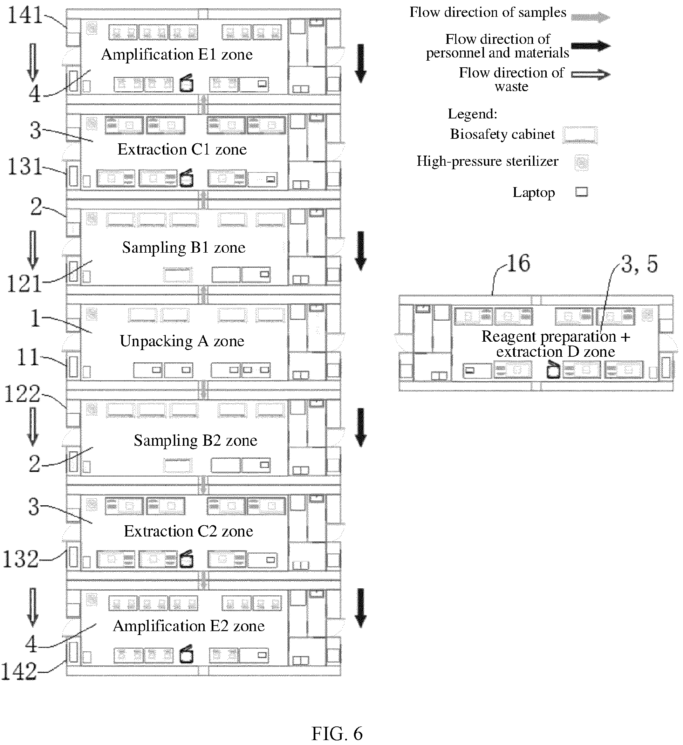

As illustrated in FIG. 6, the fourth throughput unit according to a second embodiment includes eight cabins: an unpacking cabin 11, a first sampling cabin 121, a second sampling cabin 122, a first extraction cabin 131, a second extraction cabin 132, a first amplification cabin 141, a second amplification cabin 142, and a mixing cabin 16. In view of a correspondence relationship between the cabins and the functional zones, only the sample unpacking zone 1 is provided in the unpacking cabin 11, only the sampling zone 2 is provided in the first sampling cabin 121 and the second sampling cabin 122, only the sample extraction zone 3 is provided in the first extraction cabin 131 and the second extraction cabin 132, only the nucleic acid amplification and testing zone 4 is provided in the first amplification cabin 141 and the second amplification cabin 142, and the sample extraction zone 3 and the reagent preparation zone 5 are provided in the mixing cabin 16. These eight cabins at least form two testing chains, i.e., a testing chain 1 including the unpacking A cabin 11, the sampling B1 cabin 121, the extraction C1 cabin 131, and the amplification E1 cabin 141; and a testing chain 2 including the unpacking A cabin 11, the sampling B2 cabin 122, the extraction C2 cabin 132, and the amplification E2 cabin 142. In addition, some of the operations in the extraction C1 zone and the extraction C2 zone can be performed in the extraction D zone.

The laboratory shown in FIG. 7 is formed by splicing the fourth throughput unit of FIG. 5 and is a laboratory with a testing throughput twice that of the fourth throughput unit, i.e., 100,000 per day. The types of functional zones in respective cabins may vary. For example, the sample unpacking zone 1 and the sampling zone 2 are provided in the mixing cabin 16. The laboratory includes sixteen cabins, i.e., unpacking cabins 111 to 114, sampling cabins 121 to 124, extraction cabins 131 to 134, amplification cabins 141 to 142, a reagent preparation cabin 15, and a mixing cabin 16. Only the sample unpacking zone 1 is provided in the unpacking cabins 111 to 114; only the sampling zone 2 is provided in the sampling cabins 121 to 124; only the sample extraction zone 3 is provided in the extraction cabins 131 to 134; only the nucleic acid amplification and testing zone 4 is provided in the amplification cabins 141 to 142; only the reagent preparation zone 5 is provided in the reagent preparation cabin 15; and the sample unpacking zone 1 and the sampling zone 2 are provided in the mixing cabin 16. These sixteen cabins at least form four testing chains, i.e., a testing chain 1 including the unpacking A1 cabin 111, the sampling B1 cabin 121, the extraction C1 cabin 131, and the amplification D1 cabin 141; a testing chain 2 including the unpacking A2 cabin 112, the sampling B2 cabin 122, the extraction C2 cabin 132, and the amplification D1 cabin 141; a testing chain 3 including the unpacking A3 cabin 113, the sampling B3 cabin 123, the extraction C3 cabin 133, and the amplification D2 cabin 142; and a testing chain 4 including the unpacking A4 cabin 114, the sampling B4 cabin 124, the extraction C4 cabin 134, and the amplification D2 cabin 142. In addition, some of the operations in the unpacking A1 cabin 111, the unpacking A2 cabin 112, the sampling B1 cabin 121, and the sampling B2 cabin 122 can be performed in the unpacking and sampling E zone (the mixing cabin 16).

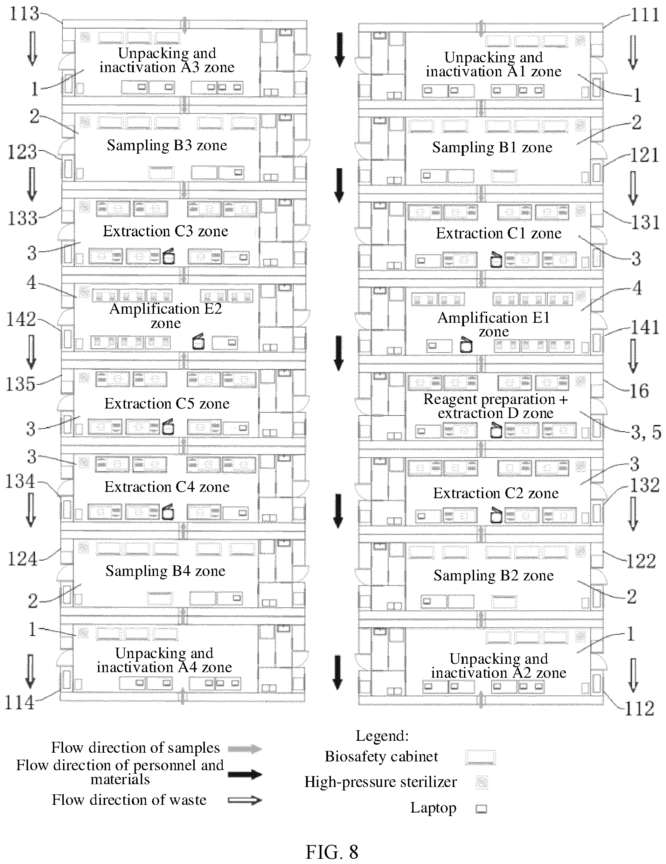

FIG. 8 is another layout of the laboratory with a testing throughput of 100,000, and the laboratory includes sixteen cabins, i.e., unpacking cabins 111 to 114, sampling cabins 121 to 124, extraction cabins 131 to 135, amplification cabins 141 to 142, and a mixing cabin 16. Only the sample unpacking zone 1 is provided in the unpacking cabins 111 to 114; only the sampling zone 2 is provided in the sampling cabins 121 to 124; only the sample extraction zone 3 is provided in the extraction cabins 131 to 135; only the nucleic acid amplification and testing zone 4 is provided in the amplification cabins 141 to 142; and the sample extraction zone 3 and the reagent preparation zone 5 are provided in the mixing cabin 16. These sixteen cabins at least form four testing chains, i.e., a testing chain 1 including the unpacking A1 cabin 111, the sampling B1 cabin 121, the extraction C1 cabin 131, and the amplification E1 cabin 141; a testing chain 2 including the unpacking A2 cabin 112, the sampling B2 cabin 122, the extraction C2 cabin 132, and the amplification E1 cabin 141; a testing chain 3 including the unpacking A3 cabin 113, the sampling B3 cabin 123, the extraction C3 cabin 133, and the amplification E2 cabin 142; and a testing chain 4 including the unpacking A4 cabin 114, the sampling B4 cabin 124, the extraction C4 cabin 134, and the amplification E2 cabin 142. In addition, some of the operations in the extraction C3 cabin 133 and the extraction C4 cabin 134 can be performed in the extraction D5 zone 135, and some of the operations in the extraction C1 cabin 131 and the extraction C2 cabin 132 can be performed in the extraction D zone.

Optionally, any one or more of the first throughput unit, the second throughput unit, the third throughput unit, or the fourth throughput unit can be combined and spliced into fast-built testing laboratories with different testing throughputs.

Optionally, a number of the at least one first throughput unit, a number of the at least one second throughput unit, a number of the at least one third throughput unit, and a number of the at least one fourth throughput unit are each one or more.

By designing the first throughput unit, the second throughput unit, the third throughput unit, and the fourth throughput unit as basic configuration units, the fast-built testing laboratory for medical testing according to the present disclosure can be formed by selecting and splicing the throughput units of different types in accordance with the requirement on the testing throughput. In this way, a laboratory management system, which can manage the testing of a daily throughput of 10,000 samples to 1 million samples, can be rapidly constructed and the safe and effective circulation of experimental data is guaranteed.

For example, the testing throughput of the first throughput unit is 10,000, the testing throughput of the second throughput unit is 20,000, the testing throughput of the third throughput unit is 30,000, and the testing throughput of the fourth throughput unit is 50,000. When a laboratory with a testing throughput of 100,000 is needed, two fourth throughput units can be directly spliced together. When a laboratory with a testing throughput of 60,000 is needed, two third throughput units can be directly spliced together, or one first throughput unit and one fourth throughput unit can be spliced together. When a laboratory with a testing throughput of 40,000 is needed, two second throughput units can be directly spliced together. When a laboratory with a testing throughput of 300,000 is needed, two fourth-throughput units can be directly spliced together to form a laboratory with a testing throughput of 100,000, and then the testing throughput of 300,000 can be reached through "one drag three" pool testing.

As the basic configuration units, the numbers of devices of the first throughput unit, the second throughput unit, the third throughput unit, and the fourth throughput unit (the testing throughputs of 10,000, 20,000, 30,000, 50,000, and 100,000) are listed in Table 2.

**[Table 2] Configuration of respective throughput units**

| Functions | Main devices | Theoretical throughput per unit of device | Max. number of key devices in one inflatable film | Max. Number of samples produced per unit of film | Min. number of key devices for standard of 10,000 samples | Min. number of key devices for standard of 20,000 samples | Min. number of key devices for standard of 30,000 samples | Min. number of key devices for standard of 50,000 samples | Min. number of key devices for standard of 100,000 samples |
|---|---|---|---|---|---|---|---|---|---|
| Number of cabins in independent structure | Inflatable film structure, tent, container, etc. | / | / | / | 3 | 4 | 5 | 8 | 16 |
| Unpacking and sample registration | Biosafety cabinet | 12000 | 4 | 48000 | 1 | 2 | 3 | 5 | 9 |
| Sampling | Biosafety cabinet | 4416 | 7 | 30912 | 3 | 5 | 8 | 12 | 23 |
| Extraction | MGISP-960 | 2945 | 7 | 20614 | 4 | 7 | 11 | 17 | 34 |
| Reagent preparation | MGSP-960 | 13252 | 7 | 92761 | 1 | 2 | 3 | 4 | 8 |
| QPCR | QPCR machine | 1104 | 48 | 52992 | 10 | 20 | 30 | 50 | 91 |

| | | | | | | | | | |
|---|---|---|---|---|---|---|---|---|---|
| Note: QPCR represents Real-time Quantitative PCR Detecting System | | | | | | | | | |

For example, in the nucleic acid testing of Covid-19, the main steps limiting the throughput are unpacking and sampling, and enough personnel is required for these two steps.

In a specific embodiment, the testing throughput of the fast-built testing laboratory can be increased through multiple-sample mixed sampling or pool testing. For example, when the testing throughput is 300,000, the testing efficiency can be enhanced through the "one drag three" solution, i.e., three samples are mixed for testing. In this way, the number of cabins for the testing throughput of 300,000 can be the same as that for a testing throughput of 100,000, but the testing capability can be increased three times. It can be seen that the testing capability of the fast-built testing laboratory can be changed by rationally designing the testing manners, and thus the fast-built testing laboratory has better flexibility.

In other words, for samples exceeding 100,000, the mixed sampling or the pool testing can be used to control the overall testing volume at about 100,000. For example, if 300,000 samples need to be tested, a "3pool1" pool testing can be adopted, i.e., certain volumes are respectively taken from 3 samples and mixed into a new tube of sample. If the test result is negative, it indicates that no virus has been detected in these 3 samples. If it is found to be positive, these 3 samples are re-tested separately to find out which sample the virus originated from.

Auxiliary functions of the fast-built testing laboratory for medical testing according to the present disclosure are introduced below.

### (I) Laboratory site selection

1) Large indoor spaces or large semi-outdoor spaces are preferred, such as large warehouses, factories, and gymnasiums.

**[Table 3] Correspondence table between testing throughput and site area size**

| Index | Daily throughput of 5,000 | Daily throughput of 10,000 | Daily throughput of 100,000 |
|---|---|---|---|
| Site area size | 18.5m×20m | 35m×20m | 54m×36m |
| Examples | Large warehouse | factory/basketball court | Gymnasium |

2) Second preferred are outdoor flat grounds, such as playgrounds of universities, middle schools, and elementary schools, airport outdoor grounds, sheltered parking lots.

3) The selected site should be convenient for entering and exiting of the flow of personnel and trucks, where a truck passage channel along the line should be 4m×4m. The site shall meet fire-fighting requirements and be equipped with fire-fighting facilities (such as heptafluoropropane).

4) A size of entry and exit logistics passage channels and a size of a freight elevator are required to be determined. A minimum passage channel size is 2.15m× 1.2m. Optionally, forklifts are allowed in or out, so as to further accelerate the transportation efficiency.

5) It is recommended that water can be supplied on site. If cannot, barreled drinking water can be used. The site should have convenient drainage, no water accumulation, and there should be public toilets nearby.

6) Not preferred are uneven outdoor terrains, wind valleys with wind above level 8, or sites with strong surface runoff in history. Preferably, a land surface seeper does not exceed 10cm after a heavy rain.

7) A minimum size of the site for a throughput of 1,000 to 5,000 is 18.5m×20m, a minimum size of a site for a throughput of 6,000 to 10,000 is a standard basketball court (35m×20m), and a minimum size of a site for a throughput of 100,000 is a standard gymnasium (54m×36m).

8) The laboratory is as close as possible to the place where the epidemic occurred, which is conducive to increase the testing efficiency and reducing logistics. For example, in the cities with an imported epidemic, the laboratory is recommended to be placed in external transportation hubs such as airports and railway stations.

9) For endogenous epidemics, epidemic-concentrated regions are selected, which is convenient for population testing and sample delivery.

10) After the epidemic is eliminated, the laboratory may be relocated.

### (II) Personnel post and responsibility allocation

The technical posts of the testing laboratory according to the present disclosure can be divided into in-cabin posts and external support posts. Through the above settings, dedicated personnel are responsible for dedicated posts, with comprehensive coverage, thereby ensuring ordered and efficient industrialized operations of the laboratory. The posts are clearly distinguished, i.e., divided into experimental groups and non-experimental groups. The requirements for personnel are diversified to ensure that personnel can be quickly trained, quickly start to work, and be quickly grouped. The personnel in key posts shall have relevant qualifications, and those in ordinary posts can obtain post permit after specific training, so as to meet the deployment of personnel in emergency. For example, the sample unpacking work requires less professionalism of personnel, and the personnel can be quickly trained to meet the requirements of unpacking work. Also, since the entire testing process is refined into individual steps, the personnel can quickly start the work after specific training. The specific functions of the posts are listed in Table 4 below.

**[Table 4] Allocation of technical posts and responsibilities in testing laboratory**

| Grouping | Group | Responsibilities |
|---|---|---|
| Non-experimental groups | Sample center | Sample receiving, registration, transfer, management, and destruction |
| | On-site group | Responsible for on-site 5S, waste transfer and sterilization |
| | Material Group | Responsible for the supply of materials on duty |
| | Co-ordination group | On-site experiment coordinator being responsible for the overall work of experimental groups on duty, and data coordinator being responsible for data statistics on duty |
| | Data analysis | Responsible for QPCR data analysis |
| | Report group | Responsible for uploading the result data to the government system |
| Experimental groups | Unpacking group | Sample unpacking, task list scheduling |
| | Sampling group | Transferring sample from tube to deep well plate |
| | Extraction group | Operating MGISP-960 to complete sample nucleic acid extraction |
| | Amplification group | Operating QPCR machine to complete sample nucleic acid amplification |
| | Reagent preparation group | Responsible for preparation and distribution of extraction reagents and QPCR reagents |

### (III) Material Supply

The details and quantities of various materials are determined by calculating the required quantity for testing. When a batch of a key material is to be changed, the performance of the batch should be confirmed in advance.

### (IV) Device performance verification

The device performance and testing methods in each cabin are determined with standard products, and the operation manual is standardized to form a standardized process.

### (V) Quality Control

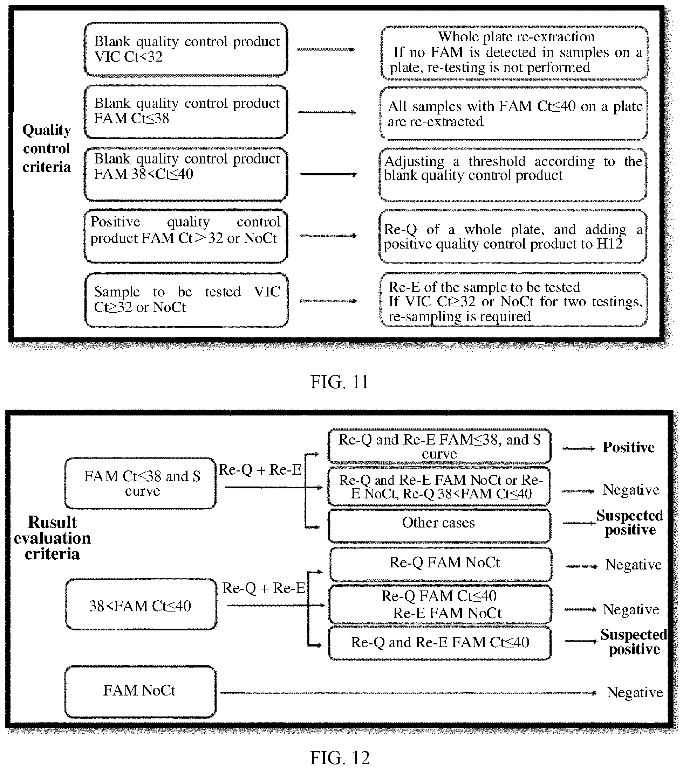

As an example, in the real-time fluorescent quantitative PCR nucleic acid testing of Covid-19, the data analysis post can ensure that the testing results meet the testing requirements, and corresponding solutions are provided for the samples failing to meet the requirements. Negative quality control: VIC Ct>32, FAM Ct>38, indicating no internal reference gene or Covid-19 RNA; positive quality control: VIC Ct<32, FAM Ct<32, indicating high expression of internal reference gene and Covid-19 RNA. Negative and positive quality control samples are fixed in middle sample holes and participate in the whole testing process in the same batch as the sample to be tested. The positive quality control sample is positive, and the 3 blank controls are all negative, which is deemed to be in control; otherwise, it is out of control and no report should be issued. The cause of the out-of-control occurrence is analyzed immediately, and the testing is performed again if necessary. The quality control standard is shown in FIG. 11, and the criteria for result evaluation are shown in FIG. 12, in which re-Q" refers to re-amplification, and "re-E" refers to re-extraction.

The fast-built testing laboratory for medical testing according to the present disclosure has the following beneficial effects:
(1) Diversity, flexibility and portability, not only suitable for the testing of Covid-19 nucleic acid samples, but also suitable for the testing of infectious viruses such as influenza A virus and influenza B virus or other products to be tested, solving the problem of medical testing resources required by different locations for public health security.
(2) By using the fast-built cabins, the testing laboratory can be rapidly constructed, the laboratory hardware is detachable and movable, and the laboratory software can be quickly copied.
(3) Through a large-scale application of fast-built testing laboratories to test Covid-19 virus, verified is a novel solution for rapidly, flexibly, and efficiently constructing and operating the laboratories in response to public health incidents.
(4) The laboratory management system can manage a large amount of data circulating in the laboratory, and can be flexibly configured and quickly implemented according to different application scenarios.
(5) The personnel training time is short, and multiple people operate together to speed up the testing.
(6) In the laboratory, multiple re-check measures are adopted, thereby having the functions of error avoidance and error prevention.
(7) Good reusability is provided, and the laboratory construction, personnel training and quality system establishment can be completed in a very short time; it is also very convenient for quick dismantling after use; and the dismantled independent structures and devices can also be transferred to other places for further use, thereby avoiding idle waste of resources.
(8) With respect to different testing throughputs, different site sizes, different fund allocations, and different professional staff allocations, corresponding solutions can be provided based on this system, so as to solve emergent public health incidents and provide the foundation for constructing a movable medical testing laboratory.

## Claims

1. A fast-built testing laboratory for medical testing, comprising a plurality of cabins, said cabins in the plurality of cabins being fast-built cabins, the plurality of cabins forming one or more testing chains, and each of the one or more testing chains comprising:
a sample unpacking zone (1);
a sampling zone (2);
a sample extraction zone (3); and
a nucleic acid amplification and testing zone (4),
wherein, for each of the one or more testing chains, extraction reagents required by the sample extraction zone (3) and amplification reagents required by the nucleic acid amplification and testing zone (4) are provided by a reagent preparation zone (5), and
wherein a cabin quantity of the plurality of cabins is adjustable to be adapted to a testing throughput of the fast-built testing laboratory,
wherein the fast-built testing laboratory further comprises a laboratory management system configured to manage sample data in the plurality of cabins,
**characterized in that** the laboratory management system comprises:
a sample center configured such, that samples are received, registered, and added with internal tracking numbers to form a package reception registration form;
an experimental center comprising the one or more testing chains, configured such, that in the experimental center, sample unpacking, sampling, sample extraction, and amplification are performed and data information generated in each process is recorded;
an analysis center configured to at least analyze data in the nucleic acid amplification and testing zone (4);
a report center configured to generate a report of a testing result of the nucleic acid amplification and testing zone (4); and
a statistics center configured to statistically obtain sample testing progresses and sample testing results.

2. The fast-built testing laboratory for medical testing according to claim 1, configured such, that
a sample obtained in the sample unpacking zone (1) is marked with a sample code, and the laboratory management system is configured to allow input of information of the sample only when the sample code is verified; or
the laboratory management system is a localized laboratory management system supporting a local working mode, or it is a cloud platform laboratory management system supporting the local working mode and a remote collaborative working mode.

3. The fast-built testing laboratory for medical testing according to claim 1, wherein the plurality of cabins comprises an unpacking cabin (11), a sampling cabin (12), an extraction cabin (13), an amplification cabin (14), and a reagent preparation cabin (15), wherein the sample unpacking zone (1) is provided in the unpacking cabin (11), the sampling zone (2) is provided in the sampling cabin (12), the sample extraction zone (3) is provided in the extraction cabin (13), the nucleic acid amplification and testing zone (4) is provided in the amplification cabin (14), and the reagent preparation zone (5) is provided in the reagent preparation cabin (15).

4. The fast-built testing laboratory for medical testing according to claim 3, wherein the plurality of cabins comprises an integrated unpacking and sampling cabin (17), wherein the sample unpacking zone (1) and/or the sampling zone (2) is provided in the integrated unpacking and sampling cabin (17), and the integrated unpacking and sampling cabin (17) is configured to supplement the unpacking cabin (11) and/or the sampling cabin (12) in quantity, and preferably, the integrated unpacking and sampling cabin (17) is configured to selectively serve as a sample unpacking zone (1) and/or a sampling zone (2) depending on a sample unpacking progress and a sampling progress as recorded in the laboratory management system.

5. The fast-built testing laboratory for medical testing according to claim 4, wherein the cabin quantity of the plurality of cabins is sixteen, and the sixteen cabins comprise: a first unpacking cabin (111), a second unpacking cabin (112), a third unpacking cabin (113), a fourth unpacking cabin (114), a first sampling cabin (121), a second sampling cabin (122), a third sampling cabin (123), a forth sampling cabin (124), a first extraction cabin (131), a second extraction cabin (132), a third extraction cabin (133), a fourth extraction cabin (134), a first amplification cabin (141), a second amplification cabin (142), a reagent preparation cabin (15) and the integrated unpacking and sampling cabin (17),
wherein the sixteen cabins form at least four testing chains: a first testing chain comprising the first unpacking cabin (111), the first sampling cabin (121), the first extraction cabin (131), and the first amplification cabin (141); a second testing chain comprising the second unpacking cabin (112), the second sampling cabin (122), the second extraction cabin (132), and the first amplification cabin (141); a third testing chain comprising the third unpacking cabin (113), the third sampling cabin (123), the third extraction cabin (133), the second amplification cabin (142); and a fourth testing chain comprising the fourth unpacking cabin (114), the fourth sampling cabin (124), the fourth extraction cabin (134), and the second amplification cabin (142),
wherein the reagent preparation cabin (15) is configured to provide extraction reagents for the first to fourth extraction cabins (131 to 134) and provide amplification reagents for the first and second amplification cabins (141, 142), and
wherein the integrated unpacking and sampling cabin (17) is configured to supplement the first to fourth unpacking cabins (111 to 114) and/or the first to fourth sampling cabins (121 to 124) in quantity.

6. The fast-built testing laboratory for medical testing according to claim 1, wherein the sample unpacking zone (1) and the sampling zone (2) are located in different cabins or in a same cabin; the sampling zone (2) and the sample extraction zone (3) are located in different cabins or in a same cabin; the sample extraction zone (3) and the reagent preparation zone (5) are located in different cabins or in a same cabin; and the nucleic acid amplification and testing zone (4) is located in a different cabin from any other zones; and/or
among the plurality of cabins, a number of cabins corresponding to zones requiring longer operation time is greater than or equal to a number of cabins corresponding to zones requiring shorter operation time; and/or
the plurality of cabins is constructed as inflatable film structures, tents, containers, or combinations thereof; and/or
two adjacent cabins of the plurality of cabins are configured to transfer materials there between through a transfer unit; and/or
the fast-built testing laboratory for medical testing further comprises a hazardous waste treatment system configured to treat medical waste generated in the plurality of cabins into non-hazardous waste; and/or
each of the plurality of cabins is a movable and fast-removable cabin.

7. The fast-built testing laboratory for medical testing according to any one of claims 1 to 6, comprising:
at least one first throughput unit each comprising three cabins of the plurality of cabins, the three cabins forming at least one of the one or more testing chains; and/or
at least one second throughput unit each comprising four cabins of the plurality of cabins, the four cabins forming at least one of the one or more testing chains; and/or
at least one third throughput unit each comprising five cabins of the plurality of cabins, the five cabins forming at least one of the one or more testing chains; and/or
at least one fourth throughput unit each comprising eight cabins of the plurality of cabins, the eight cabins forming at least one of the one or more testing chains.

8. The fast-built testing laboratory for medical testing according to claim 7, wherein the fast-built testing laboratory is provided with different testing throughputs by combining any one or more of the at least one first throughput unit, the at least one second throughput unit, the at least one third throughput unit, or the at least one fourth throughput unit.

9. The fast-built testing laboratory for medical testing according to claim 1, wherein the fast-built testing laboratory comprises one or more first throughput units, one or more second throughput units, one or more third throughput units, and one or more fourth throughput units.

10. The fast-built testing laboratory for medical testing according to claim 7, wherein the three cabins in each of the at least one first throughput unit comprise a first mixing cabin (161), a second mixing cabin (162), and an amplification cabin (14), and wherein the sample unpacking zone (1) and the sampling zone (2) are provided in the first mixing cabin (161), the sample extraction zone (3) and the reagent preparation zone (5) are provided in the second mixing cabin (162), and the nucleic acid amplification and testing zone (4) are provided in the amplification cabin (14).

11. The fast-built testing laboratory for medical testing according to claim 7, wherein the four cabins in each of the at least one second throughput unit comprise a first mixing cabin (161), a second mixing cabin (162), a third mixing cabin (163), and an amplification cabin (14), wherein the sample unpacking zone (1) and the sampling zone (2) are provided in the first mixing cabin (161), the sampling zone (2) and the sample extraction zone (3) are provided in the second mixing cabin (162), the sample extraction zone (3) and the reagent preparation zone (5) are provided in the third mixing cabin (163), and the nucleic acid amplification and testing zone (4) is provided in the amplification cabin (14).

12. The fast-built testing laboratory for medical testing according to claim 7, wherein the five cabins in each of the at least one third throughput unit comprise an unpacking cabin (11), a sampling cabin (12), an extraction cabin (13), an amplification cabin (14), and a reagent preparation cabin (15), wherein the sample unpacking zone (1) is provided in the unpacking cabin (11), the sampling zone (2) is provided in the sampling cabin (12), the sample extraction zone (3) is provided in the extraction cabin (13), the nucleic acid amplification and testing zone (4) is provided in the amplification cabin (14), and the reagent preparation zone (5) is provided in the reagent preparation cabin (15); or
wherein the five cabins in each of the at least one third throughput unit comprise an unpacking cabin (11), a sampling cabin (12), an extraction cabin (13), an amplification cabin (14), and a mixing cabin (16), wherein the sample unpacking zone (1) is provided in the unpacking cabin (11), the sampling zone (2) is provided in the sampling cabin (12), the sample extraction zone (3) is provided in the extraction cabin (13), the nucleic acid amplification and testing zone (4) is provided in the amplification cabin (14), and the sample extraction zone (3) and the reagent preparation zone (5) are provided in the mixing cabin (16).

13. The fast-built testing laboratory for medical testing according to claim 7, wherein the eight cabins in each of the at least one fourth throughput unit comprise a first unpacking cabin (111), a second unpacking cabin (112), a first sampling cabin (121), a second sampling cabin (122), a first extraction cabin (131), a second extraction cabin (132), an amplification cabin (14), and a reagent preparation cabin (15), wherein the sample unpacking zone (1) is provided in the first unpacking cabin (111) and the second unpacking cabin (112), the sampling zone (2) is provided in the first sampling cabin (121) and the second sampling cabin (122), the sample extraction zone (3) is provided in the first extraction cabin (131) and the second extraction cabin (132), the nucleic acid amplification and testing zone (4) is provided in the amplification cabin (14), and the reagent preparation zone (5) is provided in the reagent preparation cabin (15); or
wherein the eight cabins in each of the at least one fourth throughput unit comprise an unpacking cabin (11), a first sampling cabin (121), a second sampling cabin (122), a first extraction cabin (131), a second extraction cabin (132), a first amplification cabin (141), a second amplification cabin (142), and a mixing cabin (16), wherein the sample unpacking zone (1) is provided in the unpacking cabin (11), the sampling zone (2) is provided in the first sampling cabin (121) and the second sampling cabin (122), the sample extraction zone (3) is provided in the first extraction cabin (131) and the second extraction cabin (132), the nucleic acid amplification and testing zone (4) is provided in the first amplification cabin (141) and the second amplification cabin (142), and the sample extraction zone (3) and the reagent preparation zone (5) are provided in the mixing cabin (16).

## Patentansprüche

1. Schnelltestlabor für medizinische Tests, umfassend eine Vielzahl von Kabinen, wobei
die Kabinen in der Vielzahl von Kabinen Schnelltestkabinen sind, wobei die Vielzahl von Kabinen eine oder mehrere Testketten bildet und jede der einen oder der mehreren Testketten umfassend:
eine Probenentpackzone (1);
eine Probenahmezone (2);
eine Probenentnahmezone (3); und
eine Nukleinsäureamplifikations- und Testzone (4),
wobei für jede der einen oder der mehreren Testketten Entnahmereagenzien, die durch die Probenentnahmezone (3) benötigt werden, und Amplifikationsreagenzien, die durch die Nukleinsäureamplifikations- und Testzone (4) benötigt werden, durch eine Reagenzvorbereitungszone (5) bereitgestellt werden, und
wobei eine Kabinenanzahl der Vielzahl von Kabinen einstellbar ist, um an einen Testdurchsatz des Schnelltestlabors eingestellt zu werden,
wobei das Schnelltestlabor ferner ein Laborverwaltungssystem umfasst, das konfiguriert ist, um Probendaten in der Vielzahl von Kabinen zu verwalten,
**dadurch gekennzeichnet, dass** das Laborverwaltungssystem umfasst:
ein Probenzentrum, das so konfiguriert ist, dass Proben empfangen, registriert und mit internen Kontrollnummern versehen werden, um ein Paketempfangsregistrierungsformular zu erstellen;
ein Versuchszentrum, umfassend die eine oder die mehreren Testketten, das so konfiguriert ist, dass in dem Versuchszentrum das Auspacken der Proben, die Probenahme, die Probenentnahme und die Amplifikation durchgeführt werden und die in jedem Prozess erzeugten Dateninformationen aufgezeichnet werden;
ein Analysezentrum, das konfiguriert ist, um mindestens die Daten in der Nukleinsäureamplifikations- und Testzone (4) zu analysieren;
ein Berichtszentrum, das konfiguriert ist, um einen Bericht eines Testergebnisses der Nukleinsäureamplifikations- und Testzone (4) zu erzeugen; und
ein Statistikzentrum, das konfiguriert ist, um den Fortschritt der Probentests und die Ergebnisse der Probentests statistisch zu erhalten.

2. Schnelltestlabor für medizinische Tests nach Anspruch 1, das so konfiguriert ist, dass
eine in der Probenentpackzone (1) erhaltene Probe mit einem Probencode gekennzeichnet wird und das Laborverwaltungssystem konfiguriert ist, um die Eingabe von Informationen zu der Probe nur zuzulassen, wenn der Probencode verifiziert ist; oder
das Laborverwaltungssystem ein lokalisiertes Laborverwaltungssystem ist, das einen lokalen Arbeitsmodus unterstützt, oder es ein Cloud-Plattform-Laborverwaltungssystem ist, das den lokalen Arbeitsmodus und einen entfernten Zusammenarbeitsmodus unterstützt.

3. Schnelltestlabor für medizinische Tests nach Anspruch 1, wobei die Vielzahl von Kabinen eine Entpackkabine (11), eine Probenahmekabine (12), eine Entnahmekabine (13), eine Amplifikationskabine (14) und eine Reagenzvorbereitungskabine (15) umfasst, wobei die Probenentpackzone (1) in der Entpackkabine (11) bereitgestellt ist, und die Probenahmezone (2) in der Probenahmekabine (12) bereitgestellt ist, die Probeentnahmezone (3) in der Entnahmekabine (13) bereitgestellt ist, die Nukleinsäureamplifikations- und Testzone (4) in der Amplifikationskabine (14) bereitgestellt ist und die
Reagenzvorbereitungszone (5) in der Reagenzvorbereitungskabine (15) bereitgestellt ist.

4. Schnelltestlabor für medizinische Tests nach Anspruch 3, wobei die Vielzahl von Kabinen eine integrierte Entpack- und Probenahmekabine (17) umfasst, wobei die Probenentpackzone (1) und/oder die Probenahmezone (2) in der integrierten Entpack- und Probenahmekabine (17) bereitgestellt ist, und die integrierte Entpack- und Probenahmekabine (17) konfiguriert ist, um die Entpackkabine (11) und/oder die Probenahmekabine (12) quantitativ zu ergänzen, und vorzugsweise die integrierte Entpack- und Probenahmekabine (17) konfiguriert ist, um wahlweise als eine Probenentpackzone (1) und/oder eine Probenahmezone (2) zu fungieren, abhängig von einem Probenentpackfortschritt und einem Probenahmefortschritt, wie sie in dem Laborverwaltungssystem aufgezeichnet sind.

5. Schnelltestlabor für medizinische Tests nach Anspruch 4, wobei die Kabinenanzahl der Vielzahl von Kabinen sechzehn beträgt und die sechzehn Kabinen umfassen: eine erste Entpackkabine (111), eine zweite Entpackkabine (112), eine dritte Entpackkabine (113), eine vierte Entpackkabine (114), eine erste Probenahmekabine (121), eine zweite Probenahmekabine (122), eine dritte Probenahmekabine (123), eine vierte Probenahmekabine (124), eine erste Entnahmekabine (131), eine zweite Entnahmekabine (132), eine dritte Entnahmekabine (133), eine vierte Entnahmekabine (134), eine erste Amplifikationskabine (141), eine zweite Amplifikationskabine (142), eine Reagenzvorbereitungskabine (15) und die integrierte Entpack- und Probenahmekabine (17),
wobei die sechzehn Kabinen mindestens vier Testketten bilden: eine erste Testkette, umfassend die erste Entpackkabine (111), die erste Probenahmekabine (121), die erste Entnahmekabine (131) und die erste Amplifikationskabine (141); eine zweite Testkette, umfassend die zweite Entpackkabine (112), die zweite Probenahmekabine (122), die zweite Entnahmekabine (132) und die erste Amplifikationskabine (141); eine dritte Testkette, umfassend die dritte Entpackkabine (113), die dritte Probenahmekabine (123), die dritte Entnahmekabine (133), die zweite Amplifikationskabine (142); und eine vierte Testkette, umfassend die vierte Entpackkabine (114), die vierte Probenahmekabine (124), die vierte Entnahmekabine (134), die zweite Amplifikationskabine (142),
wobei die Reagenzvorbereitungskabine (15) konfiguriert ist, um Entnahmereagenzien für die erste bis vierte Entnahmekabine (131 bis 134) bereitzustellen und Amplifikationsreagenzien für die erste und die zweite Amplifikationskabine (141, 142) bereitzustellen, und
wobei die integrierte Entpack- und Probenahmekabine (17) konfiguriert ist, um die erste bis vierte Entpackkabine (111 bis 114) und/oder die erste bis vierte Probenahmekabine (121 bis 124) quanitativ zu ergänzen.

6. Schnelltestlabor für medizinische Tests nach Anspruch 1, wobei sich der Probenentpackzone (1) und die Probenahmezone (2) in verschiedenen Kabinen oder in einer gleichen Kabine befinden; die Probenahmezone (2) und die Probenentnahmezone (3) in verschiedenen Kabinen oder in einer gleichen Kabine befinden; die Probenentnahmezone (3) und die Reagenzienvorbereitungszone (5) in verschiedenen Kabinen oder in einer gleichen Kabine befinden; und sich die Nukleinsäureamplifikations- und Testzone (4) in einer anderen Kabine als alle anderen Zonen befindet; und/oder
unter der Vielzahl von Kabinen eine Anzahl von Kabinen entsprechend den Zonen, die eine längere Vorgangsdauer erfordern, größer als oder gleich einer Anzahl von Kabinen entsprechend den Zonen ist, die eine kürzere Vorgangsdauer erfordern; und/oder
die Vielzahl von Kabinen als aufblasbare Folienstrukturen, Zelte, Behältnisse oder Kombinationen davon aufgebaut ist; und/oder
zwei benachbarte Kabinen der Vielzahl von Kabinen konfiguriert sind, um Materialien zwischen diesen durch eine Übergabeeinheit zu überführen; und/oder
das Schnelltestlabor für medizinische Tests ferner ein System für eine Aufbereitung gefährlicher Abfälle umfasst, das konfiguriert ist, um die in der Vielzahl der Kabinen erzeugten medizinischen Abfälle in nicht gefährliche Abfälle aufzubereiten; und/oder
jede der Vielzahl von Kabinen eine bewegbare und schnell entfernbare Kabine ist.

7. Schnelltestlabor für medizinische Tests nach einem der Ansprüche 1 bis 6, umfassend:
mindestens eine erste Durchsatzeinheit, umfassend jeweils drei Kabinen der Vielzahl von Kabinen, wobei die drei Kabinen mindestens eine der einen oder der mehreren Testketten bilden; und/oder
mindestens eine zweite Durchsatzeinheit, umfassend jeweils vier Kabinen der Vielzahl von Kabinen, wobei die vier Kabinen mindestens eine der einen oder der mehreren Testketten bilden; und/oder
mindestens eine dritte Durchsatzeinheit, umfassend jeweils fünf Kabinen der Vielzahl von Kabinen, wobei die fünf Kabinen mindestens eine der einen oder der mehreren Testketten bilden; und/oder
mindestens eine vierte Durchsatzeinheit, umfassend jeweils acht Kabinen der Vielzahl von Kabinen, wobei die acht Kabinen mindestens eine der einen oder der mehreren Testketten bilden.

8. Schnelltestlabor für medizinische Tests nach Anspruch 7, wobei das Schnelltestlabor durch Kombinieren von einer beliebigen von einer oder mehreren der mindestens einen ersten Durchsatzeinheit, der mindestens einen zweiten Durchsatzeinheit, der mindestens einen dritten Durchsatzeinheit oder der mindestens einen vierten Durchsatzeinheit mit verschiedenen Testdurchsätzen versehen ist.

9. Schnelltestlabor für medizinische Tests nach Anspruch 1, wobei das Schnelltestlabor eine oder mehrere erste Durchsatzeinheiten, eine oder mehrere zweite Durchsatzeinheiten, eine oder mehrere dritte Durchsatzeinheiten und eine oder mehrere vierte Durchsatzeinheiten umfasst.

10. Schnelltestlabor für medizinische Tests nach Anspruch 7, wobei die drei Kabinen in jeder der mindestens einen ersten Durchsatzeinheit eine erste Mischkabine (161), eine zweite Mischkabine (162) und eine Amplifikationskabine (14) umfassen, und wobei die Probenentpackzone (1) und die Probenahmezone (2) in der ersten Mischkabine (161) bereitgestellt sind, die Probenentnahmezone (3) und die Reagenzvorbereitungszone (5) in der zweiten Mischkabine (162) bereitgestellt sind und die Nukleinsäureamplifikations- und Testzone (4) in der Amplifikationskabine (14) bereitgestellt sind.

11. Schnelltestlabor für medizinische Tests nach Anspruch 7, wobei die vier Kabinen in jeder der mindestens einen zweiten Durchsatzeinheit eine erste Mischkabine (161), eine zweite Mischkabine (162) und eine dritte Mischkabine (163) und eine Amplifikationskabine (14) umfassen, wobei die Probenentpackzone (1) und die Probenahmezone (2) in der ersten Mischkabine (161) bereitgestellt sind, die Probenahmezone (2) und die Probenentnahmezone (3) in der zweiten Mischkabine (162) bereitgestellt sind, die Probenentnahmezone (3) und die Reagenzvorbereitungszone (5) in der dritten Mischkabine (163) bereitgestellt sind und die Nukleinsäureamplifikations- und Testzone (4) in der Amplifikationskabine (14) bereitgestellt sind.

12. Schnelltestlabor für medizinische Tests nach Anspruch 7, wobei die fünf Kabinen in jeder der mindestens einen dritten Durchsatzeinheit eine Entpackkabine (11), eine Probenahmekabine (12), eine Entnahmekabine (13), eine Amplifikationskabine (14) und eine Reagenzienvorbereitungskabine (15) umfassen, wobei die Probenentpackzone (1) in der Entpackkabine (11) bereitgestellt ist, die Probenahmezone (2) in der Probenahmekabine (12) bereitgestellt ist, die Probenentnahmezone (3) in der Entnahmekabine (13) bereitgestellt ist, die Nukleinsäureamplifikations- und Testzone (4) in der Amplifikationskabine (14) bereitgestellt ist und die Reagenzvorbereitungszone (5) in der Reagenzvorbereitungskabine (15) bereitgestellt ist; oder
wobei die fünf Kabinen in jeder der mindestens einen dritten Durchsatzeinheit eine Entpackkabine (11), eine Probenahmekabine (12), eine Entnahmekabine (13), eine Amplifikationskabine (14) und eine Mischkabine (16) umfassen, wobei die Probenentpackzone (1) in der Entpackkabine (11) bereitgestellt ist, die Probenahmezone (2) in der Probenahmekabine (12) bereitgestellt ist, die Probenentnahmezone (3) in der Entnahmekabine (13) bereitgestellt ist, die Nukleinsäureamplifikations- und Testzone (4) in der Amplifikationskabine (14) bereitgestellt ist und die Probenentnahmezone (3) und die Reagenzvorbereitungszone (5) in der Mischkabine (16) bereitgestellt sind.

13. Schnelltestlabor für medizinische Tests nach Anspruch 7, wobei die acht Kabinen in jeder der mindestens einen vierten Durchsatzeinheit eine erste Entpackkabine (111), eine zweite Entpackkabine (112), eine erste Probenahmekabine (121), eine zweite Probenahmekabine (122), eine erste Entnahmekabine (131), eine zweite Entnahmekabine (132), eine Amplifikationskabine (14) und eine Reagenzienvorbereitungskabine (15) umfassen, wobei die Probenentpackzone (1) in der ersten Entpackkabine (111) und der zweiten Entpackkabine (112) bereitgestellt ist, die Probenahmezone (2) in der ersten Probenahmekabine (121) und der zweiten Probenahmekabine (122) bereitgestellt ist, die Probenentnahmezone (3) in der ersten Entnahmekabine (131) und der zweiten Entnahmekabine (132) bereitgestellt ist, die Nukleinsäureamplifikations- und Testzone (4) in der Amplifikationskabine (14) bereitgestellt ist, und die Reagenzvorbereitungszone (5) in der Reagenzvorbereitungskabine (15) bereitgestellt ist; oder
wobei die acht Kabinen in jeder der mindestens einen vierten Durchsatzeinheit eine Entpackkabine (11), eine erste Probenahmekabine (121), eine zweite Probenahmekabine (122), eine erste Entnahmekabine (131), eine zweite Entnahmekabine (132), eine erste Amplifikationskabine (141), eine zweite Amplifikationskabine (142) und eine Mischkabine (16) umfassen, wobei die Probenentpackzone (1) in der Entpackkabine (11) bereitgestellt ist, die Probenahmezone (2) in der ersten Probenahmekabine (121) und der zweiten Probenahmekabine (122) bereitgestellt ist, die Probenentnahmezone (3) in der ersten Entnahmekabine (131) und der zweiten Entnahmekabine (132) bereitgestellt ist, die Nukleinsäureamplifikations- und Testzone (4) in der ersten Amplifikationskabine (141) und der zweiten Amplifikationskabine (142) bereitgestellt ist, und die Probenentnahmezone (3) und die Reagenzvorbereitungszone (5) in der Mischkabine (16) bereitgestellt sind.

## Revendications

1. Laboratoire d'essais à construction rapide pour essais médicaux, comprenant une pluralité de cabines, lesdites cabines dans la pluralité de cabines étant des cabines à construction rapide, la pluralité de cabines formant une ou plusieurs chaînes d'essais, et chacune de la une ou des plusieurs chaînes d'essais comprenant :
une zone de déballage d'échantillons (1) ;
une zone d'échantillonnage (2) ;
une zone d'extraction d'échantillons (3) ; et
une zone d'amplification et d'essais des acides nucléiques (4),
dans lequel, pour chacune de la une ou des plusieurs chaînes d'essais, les réactifs d'extraction requis par la zone d'extraction d'échantillons (3) et les réactifs d'amplification requis par la zone d'amplification et d'essais des acides nucléiques (4) sont fournis par une zone de préparation des réactifs (5), et
dans lequel un nombre de cabines de la pluralité de cabines est réglable pour s'adapter à un débit d'essais du laboratoire d'essais à construction rapide,
dans lequel le laboratoire d'essais à construction rapide comprend en outre un système de gestion de laboratoire configuré pour gérer les données d'échantillon dans la pluralité de cabines,
**caractérisé en ce que** le système de gestion de laboratoire comprend :
un centre d'échantillons configuré de manière à ce que les échantillons soient reçus, enregistrés et ajoutés avec des numéros de suivi internes pour former un formulaire d'enregistrement de réception de colis ;
un centre d'expérimentation comprenant la une ou plusieurs chaînes d'essais, configuré de manière à ce que, dans le centre d'expérimentation, le déballage d'échantillons, l'échantillonnage, l'extraction d'échantillons et l'amplification soient réalisés, et que les informations de données générées au cours de chaque processus soient enregistrées ;
un centre d'analyse configuré pour, au moins, analyser les données dans la zone d'amplification et d'essais des acides nucléiques (4) ;
un centre de rapport configuré pour générer un rapport sur un résultat d'essais de la zone d'amplification et d'essais des acides nucléiques (4) ; et
un centre de statistiques configuré pour obtenir des statistiques sur l'état d'avancement et les résultats d'essais d'échantillons.

2. Laboratoire d'essais à construction rapide pour essais médicaux selon la revendication 1, configuré de manière à ce que
un échantillon obtenu dans la zone de déballage d'échantillons (1) soit marqué avec un code d'échantillon, et le système de gestion de laboratoire soit configuré pour n'autoriser la saisie d'informations sur l'échantillon que si le code d'échantillon est vérifié ; ou
le système de gestion de laboratoire soit un système de gestion de laboratoire localisé prenant en charge un mode de travail local, ou soit un système de gestion de laboratoire sur plateforme en nuage prenant en charge le mode de travail local et un mode de travail collaboratif à distance.

3. Laboratoire d'essais à construction rapide pour essais médicaux selon la revendication 1, dans lequel la pluralité de cabines comprend une cabine de déballage (11), une cabine d'échantillonnage (12), une cabine d'extraction (13), une cabine d'amplification (14) et une cabine de préparation des réactifs (15), dans lequel la zone de déballage d'échantillon (1) est fournie dans la cabine de déballage (11), la zone d'échantillonnage (2) est fournie dans la cabine d'échantillonnage (12), la zone d'extraction d'échantillon (3) est fournie dans la cabine d'extraction (13), la zone d'amplification et d'essais des acides nucléiques (4) est fournie dans la cabine d'amplification (14), et la zone de préparation des réactifs (5) est fournie dans la cabine de préparation des réactifs (15).

4. Laboratoire d'essais à construction rapide pour essais médicaux selon la revendication 3, dans lequel la pluralité de cabines comprend une cabine de déballage et d'échantillonnage intégrée (17), dans lequel la zone de déballage d'échantillons (1) et/ou la zone d'échantillonnage (2) est fournie dans la cabine de déballage et d'échantillonnage intégrée (17), et la cabine de déballage et d'échantillonnage intégrée (17) est configurée pour compléter la cabine de déballage (11) et/ou la cabine d'échantillonnage (12) en quantité, et de préférence, la cabine de déballage et d'échantillonnage intégrée (17) est configurée pour servir sélectivement d'une zone de déballage d'échantillon (1) et/ou d'une zone d'échantillonnage (2) en fonction d'un état d'avancement du déballage d'échantillons et d'un état d'avancement d'échantillonnage tels qu'enregistrés dans le système de gestion de laboratoire.

5. Laboratoire d'essais à construction rapide pour essais médicaux selon la revendication 4, dans lequel la quantité de cabines de la pluralité de cabines est de seize, et les seize cabines comprennent : une première cabine de déballage (111), une deuxième cabine de déballage (112), une troisième cabine de déballage (113), une quatrième cabine de déballage (114), une première cabine d'échantillonnage (121), une deuxième cabine d'échantillonnage (122), une troisième cabine d'échantillonnage (123), une quatrième cabine d'échantillonnage (124), une première cabine d'extraction (131), une deuxième cabine d'extraction (132), une troisième cabine d'extraction (133), une quatrième cabine d'extraction (134), une première cabine d'amplification (141), une seconde cabine d'amplification (142), une cabine de préparation des réactifs (15) et la cabine de déballage et d'échantillonnage intégrée (17),
dans lequel les seize cabines forment au moins quatre chaînes d'essais : une première chaîne d'essais comprenant la première cabine de déballage (111), la première cabine d'échantillonnage (121), la première cabine d'extraction (131) et la première cabine d'amplification (141) ; une deuxième chaîne d'essais comprenant la deuxième cabine de déballage (112), la deuxième cabine d'échantillonnage (122), la deuxième cabine d'extraction (132) et la première cabine d'amplification (141) ; une troisième chaîne d'essais comprenant la troisième cabine de déballage (113), la troisième cabine d'échantillonnage (123), la troisième cabine d'extraction (133), la seconde cabine d'amplification (142) ; et une quatrième chaîne d'essais comprenant la quatrième cabine de déballage (114), la quatrième cabine d'échantillonnage (124), la quatrième cabine d'extraction (134), et la seconde cabine d'amplification (142),
dans lequel la cabine de préparation des réactifs (15) est configurée pour fournir des réactifs d'extraction de la première à la quatrième cabine d'extraction (131 à 134) et des réactifs d'amplification aux première et seconde cabines d'amplification (141, 142), et
dans lequel la cabine de déballage et d'échantillonnage intégrée (17) est configurée pour compléter les cabines de déballage (111 à 114), et/ou de la première à la quatrième cabine d'échantillonnage (121 à 124) en quantité.

6. Laboratoire d'essais à construction rapide pour essais médicaux selon la revendication 1, dans lequel la zone de déballage d'échantillon (1) et la zone d'échantillonnage (2) sont situées dans des cabines différentes ou dans une même cabine ; la zone d'échantillonnage (2) et la zone d'extraction d'échantillons (3) sont situées dans des cabines différentes ou dans une même cabine ; la zone d'extraction d'échantillons (3) et la zone de préparation des réactifs (5) sont situées dans des cabines différentes ou dans une même cabine ; et la zone d'amplification et d'essais des acides nucléiques (4) est située dans une cabine différente de toutes les autres zones ; et/ou
parmi la pluralité de cabines, un nombre de cabines correspondant à des zones nécessitant un temps de fonctionnement plus long est supérieur ou égal à un nombre de cabines correspondant à des zones nécessitant un temps de fonctionnement plus court ; et/ou
la pluralité de cabines est constituée de structures de films gonflables, de tentes, de conteneurs ou de combinaisons de ceux-ci ; et/ou
deux cabines adjacentes de la pluralité de cabines sont configurées pour transférer des matériaux entre elles par l'intermédiaire d'une unité de transfert ; et/ou
le laboratoire d'essais à construction rapide pour essais médicaux comprend en outre un système de traitement des déchets dangereux configuré pour traiter les déchets médicaux générés dans la pluralité de cabines et les transformer en déchets non dangereux ; et/ou
chacune de la pluralité de cabines est une cabine mobile et rapidement amovible.

7. Laboratoire d'essais à construction rapide pour essais médicaux selon l'une quelconque des revendications 1 à 6, comprenant :
au moins une première unité de débit comprenant chacune trois cabines de la pluralité de cabines, les trois cabines formant au moins une de la une ou de plusieurs chaînes d'essais ; et/ou
au moins une deuxième unité de débit comprenant chacune quatre cabines de la pluralité de cabines, les quatre cabines formant au moins une de la une ou de plusieurs chaînes d'essais ; et/ou
au moins une troisième unité de débit comprenant chacune cinq de la pluralité de cabines, les cinq cabines formant au moins une de la une ou de plusieurs chaînes d'essais ; et/ou
au moins une quatrième unité de débit comprenant chacune huit cabines de la pluralité de cabines, les huit cabines formant au moins une de la une ou de plusieurs chaînes d'essais.

8. Laboratoire d'essais à construction rapide pour essais médicaux selon la revendication 7, dans lequel le laboratoire d'essais à construction rapide pour essais médicaux est fourni avec différents débits d'essais en combinant l'une quelconque ou plusieurs de la au moins une première unité de débit, la au moins une deuxième unité de débit, la au moins une troisième unité de débit, ou la au moins une quatrième unité de débit.

9. Laboratoire d'essais à construction rapide pour essais médicaux selon la revendication 1, dans lequel le laboratoire d'essais à construction rapide comprend une ou plusieurs premières unités de débit, une ou plusieurs deuxièmes unités de débit, une ou plusieurs troisièmes unités de débit, et une ou plusieurs quatrièmes unités de débit.

10. Laboratoire d'essais à construction rapide pour essais médicaux selon la revendication 7, dans lequel les trois cabines dans chacune de la au moins une première unité de débit comprennent une première cabine de mélange (161), une deuxième cabine de mélange (162), et une cabine d'amplification (14), et dans lequel la zone de déballage d'échantillons (1) et la zone d'échantillonnage (2) sont fournies dans la première cabine de mélange (161), la zone d'extraction d'échantillons (3) et la zone de préparation des réactifs (5) sont fournies dans la deuxième cabine de mélange (162), et la zone d'amplification et d'essais des acides nucléiques (4) est fournie dans la cabine d'amplification (14) .

11. Laboratoire d'essais à construction rapide pour essais médicaux selon la revendication 7, dans lequel les quatre cabines dans chacune de la au moins une deuxième unité de débit comprennent une première cabine de mélange (161), une deuxième cabine de mélange (162), une troisième cabine de mélange (163), et une cabine d'amplification (14), dans lequel la zone de déballage d'échantillons (1) et la zone d'échantillonnage (2) sont fournies dans la première cabine de mélange (161), la zone d'échantillonnage (2) et la zone d'extraction d'échantillons (3) sont fournies dans la deuxième cabine de mélange (162), la zone d'extraction d'échantillon (3) et la zone de préparation des réactifs (5) sont fournies dans la troisième cabine de mélange (163), et la zone d'amplification et d'essais des acides nucléiques (4) est fournie dans la cabine d'amplification (14).

12. Laboratoire d'essais à construction rapide pour essais médicaux selon la revendication 7, dans lequel les cinq cabines dans chacune de la au moins une troisième unité de débit comprennent une cabine de déballage (11), une cabine d'échantillonnage (12), une cabine d'extraction (13), une cabine d'amplification (14) et une cabine de préparation des réactifs (15), dans lequel la zone de déballage d'échantillons (1) est fournie dans la cabine de déballage (11), la zone d'échantillonnage (2) est fournie dans la cabine d'échantillonnage (12), la zone d'extraction d'échantillons (3) est fournie dans la cabine d'extraction (13), la zone d'amplification et d'essais des acides nucléiques (4) est fournie dans la cabine d'amplification (14), et la zone de préparation des réactifs (5) est fournie dans la cabine de préparation des réactifs (15) ; ou
dans lequel les cinq cabines de chacune de la au moins une troisième unité de débit comprennent une cabine de déballage (11), une cabine d'échantillonnage (12), une cabine d'extraction (13), une cabine d'amplification (14) et une cabine de mélange (16), dans lequel la zone de déballage d'échantillons (1) est fournie dans la cabine de déballage (11), la zone d'échantillonnage (2) est fournie dans la cabine d'échantillonnage (12), la zone d'extraction d'échantillons (3) est fournie dans la cabine d'extraction (13), la zone d'amplification et d'essais des acides nucléiques (4) est fournie dans la cabine d'amplification (14), et la zone d'extraction d'échantillons (3) et la zone de préparation des réactifs (5) sont fournies dans la cabine de mélange (16).

13. Laboratoire d'essais à construction rapide pour essais médicaux selon la revendication 7, dans lequel les huit cabines de chacune de la au moins une quatrième unité de débit comprennent une première cabine de déballage (111), une deuxième cabine de déballage (112), une première cabine d'échantillonnage (121), une deuxième cabine d'échantillonnage (122), une première cabine d'extraction (131), une deuxième cabine d'extraction (132), une cabine d'amplification (14), et une cabine de préparation des réactifs (15), dans lequel la zone de déballage d'échantillon (1) est fournie dans la première cabine de déballage (111) et la deuxième cabine de déballage (112), la zone d'échantillonnage (2) est fournie dans la première cabine d'échantillonnage (121) et la deuxième cabine d'échantillonnage (122), la zone d'extraction d'échantillons (3) est fournie dans la première cabine d'extraction (131) et la deuxième cabine d'extraction (132), la zone d'amplification et d'essais des acides nucléiques (4) est fournie dans la cabine d'amplification (14), et la zone de préparation des réactifs (5) est fournie dans la cabine de préparation des réactifs (15) ; ou
dans lequel les huit cabines de chacune de la au moins une quatrième unité de débit comprennent une cabine de déballage (11), une première cabine d'échantillonnage (121), une deuxième cabine d'échantillonnage (122), une première cabine d'extraction (131), une deuxième cabine d'extraction (132), une première cabine d'amplification (141), une seconde cabine d'amplification (142), et une cabine de mélange (16), dans lequel la zone de déballage d'échantillons (1) est fournie dans la cabine de déballage (11), la zone d'échantillonnage (2) est fournie dans la première
cabine d'échantillonnage (121) et la deuxième cabine d'échantillonnage (122), la zone d'extraction d'échantillon (3) est fournie dans la première cabine d'extraction (131) et la deuxième cabine d'extraction (132), la zone d'amplification et d'essais des acides nucléiques (4) est fournie dans la première cabine d'amplification (141) et la seconde cabine d'amplification (142), et la zone d'extraction d'échantillons (3) et la zone de préparation des réactifs (5) sont fournies dans la cabine de mélange (16).
